(19) Europäisches Patentamt
European Patent Office
Office européen des brevets

(11) EP 4 541 837 A1

# (12) EUROPEAN PATENT APPLICATION

(43) Date of publication:
**23.04.2025 Bulletin 2025/17**

(21) Application number: **23204802.5**

(22) Date of filing: **20.10.2023**

(51) International Patent Classification (IPC):
***C08G 71/04** (2006.01)* ***A61F 2/06** (2013.01)*
***A61F 2/24** (2006.01)* ***B33Y 70/00** (2020.01)*

(52) Cooperative Patent Classification (CPC):
(C-Sets available)
**C08G 71/04; A61L 29/06; A61L 31/06; B33Y 70/00**
(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Université de Liège**
**4000 Liège (BE)**

(72) Inventors:
- **OURY, Cécile**
  **4000 Liège (BE)**
- **JEROME, Christine**
  **4000 Liège (BE)**
- **MELO, Sofia**
  **4000 Liège (BE)**
- **PIERRARD, Anna**
  **4000 Liège (BE)**
- **DETREMBLEUR, Christophe**
  **4000 Liège (BE)**
- **LANCELLOTTI, Patrizio**
  **4000 Liège (BE)**

(74) Representative: **V.O.**
**P.O. Box 87930**
**2508 DH Den Haag (NL)**

## (54) POLYOXAZOLIDONE DERIVATIVES AND THEIR USE IN MEDICAL DEVICES

(57) The invention provides poly(hydroxy oxazolidone) compounds (PHOx) corresponding to following formula (I) or formula (II):

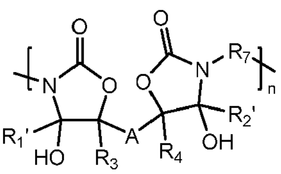
(I)

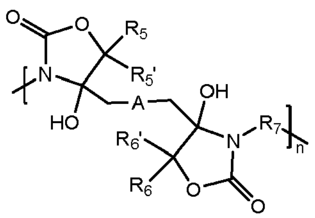
(II)

wherein
$R_{1'}$, $R_{2'}$, independently of one another each denote a C1 to C20 alkyl group, preferably a C1 to C10 alkyl group, more preferably a C1 to C3 alkyl group, most preferably a methyl group, and
- $R_3$, $R_4$ independently of one another each denote hydrogen or a C1 to C20 alkyl group, preferably a C1 to C10 alkyl group, more preferably a C1 to C3 alkyl group, most preferably a methyl group, and
- $R_5$, $R_{5'}$, $R_6$, $R_{6'}$, independently of one another each denote hydrogen or a C1 to C20 alkyl group, preferably a C1 to C10 alkyl group, more preferably a C1 to C3 alkyl group, most preferably a methyl group, or an aryl group, preferably a phenyl group, and
- A is an organic linking group selected from a linear, branched, saturated, unsaturated, cyclic and/or non-cyclic aliphatic, aromatic or araliphatic hydrocarbon group, preferably having 1 to 20, more preferably 1 to 12, most preferably 1 to 8 carbon atoms and optionally further containing one or more functional groups selected from ether, ester, amide, amine, carboxyl, sulfur, halogen and/or urethane, and
- Optionally A, $R_3$ and $R_4$ in formula (I) are linked to each other to form a cyclic structure, and
- Optionally $R_5$ and $R_{5'}$, and/or $R_6$ and $R_{6'}$ in formula (II) are respectively linked to each other to form a cyclic structure, and
- $R_7$ is a polymeric chain selected from a polysiloxane or a polyether, and
- n is an integer from 2 to 100, preferably 2 to 50, more preferably 2 to 25, most preferably 2 to 15.

EP 4 541 837 A1

The invention further provides a process for forming the PHOx compounds of the invention and poly(alkylidene oxazolidone) compounds (POx) obtained by dehydrating the PHOx compounds of the invention.

(52) Cooperative Patent Classification (CPC): (Cont.)

C-Sets
**A61L 29/06, C08L 83/04;**
**A61L 31/06, C08L 83/04**

**Description**

TECHNICAL FIELD OF THE INVENTION

The present invention relates to formulations for poly(oxazolidone) derivatives, to the process for obtaining said poly(oxazolidone) derivatives and to the use of said poly(oxazolidone) derivatives in medical devices.

BACKGROUND OF THE INVENTION

Medical devices (MDs) and biomaterial implants are clinically used in a variety of applications, and their performance is critical to a patient's overall health and quality of life. However, implantable blood-contacting MDs are subject to thrombosis and/or infection. Foreign material in contact with blood may trigger host inflammatory reactions, plasma protein and platelet adhesion, and contact pathway activation of coagulation cascade. In addition, the surface of MDs can be colonized by bacteria or fungi and eventually cause a local or a systemic infection. Such colonization can happen at the moment of implantation or long after implantation (haematogeneous infection). The elimination of the infection is highly challenging due to bacteria biofilm formation on the surface of the device. Prompt treatment of patients with anti-thrombotic agents or antibiotics is rarely effective and the removal of the MD is often the sole solution.

Moreover, complications like bleeding (with anti-thrombotic treatment) and antibiotic resistance (with antibiotic treatment) can arise. Therefore, in view of a MD implantation, the best management approach is prevention.

Biocompatibility refers to whether the implant material or its degradation products, if any, initiate adverse tissue responses in the host, or conversely, whether deleterious changes in the chemical, physical, or mechanical properties of the implant material are caused by the host environment. The term "hemocompatibility" refers to biocompatibility issues related with implantation in the cardiovascular system, such as any toxicity of implant materials to red blood cells or tissues contacted by the material.

Catheters are among the most implanted blood-contacting MDs for which there is a need for such improvements. Indeed, thrombotic and infectious complications linked to the use of catheters often occur (thrombosis: in up to 66%; infection: in up to 8% of catheter implantations), which result in longer hospitalization stay, higher health care costs, or even death. Catheters are thin tubes made mainly of polyurethane (PU) but also other medical grade plastics, like silicon rubber, polyvinyl chloride, or nylon, which are inserted in the patient's body cavity, duct or blood vessel to deliver medications or to drain bodily fluids for therapeutic, diagnostic and/or monitoring purposes. Catheters have broad clinical applications including cardiovascular, urological, gastrointestinal, neurovascular and ophthalmic. In high demand are peripheral and central venous catheters, which are intravascular catheters to ensure safe infusion of fluids or medications for a short- or long-term use (months to years).

Other blood-contacting MDs are prosthetic heart valves. Due to steadily increasing prevalence of heart valve diseases, it has been estimated that by the year 2050, over 1 million patients will require a heart valve replacement. Two major types of prosthetic heart valves exist: mechanical and bioprosthetic. Mechanical prostheses are mainly made of pyrolytic carbon combined with metallic and polymeric components. Mechanical prosthetic heart valves are nevertheless associated with thrombogenic issues. The incidence of prosthetic valve thrombosis has been reported to average 0.2% per patient-year after aortic and 1.8% per patient-year after mitral valve replacement, within a broad range from less than 0.1 to more than 6% per patient-year. The risk for prosthetic valve thrombosis appears to be twice as high in mitral as in aortic valve replacement, and is more than three times higher for a tricuspid valve prosthesis.

Bioprostheses, made of porcine valves or bovine pericardium, are less thrombogenic but they are prone to deterioration. The available prosthetic valve options that exist today have therefore several limitations. In addition, the costs and materials required to source and process biological tissue limit the widespread use of bioprosthetic valves in low-resource settings.

Early attempts to develop polymeric heart valves were unsuccessful largely due to a limited ability to modify polymeric compounds to meet the needs of an ideal heart valve substitute. Various materials have been investigated as candidates for polymeric heart valves, including poly(tetrafluoroethylene)s, polysiloxanes, poly(styrene-b-isobutylene-b-styrene), and polyurethanes. However, these polymers have limited hemo-compatibility or calcify and/or degenerate and/or weaken over time.

As such there is a need to develop a biocompatible material that is suitable for use in medical devices and would solve one or more of the above-mentioned problems.

SUMMARY

According to a first aspect, the invention provides poly(hydroxy oxazolidone) compounds (PHOx) corresponding to following formula (I) or formula (II):

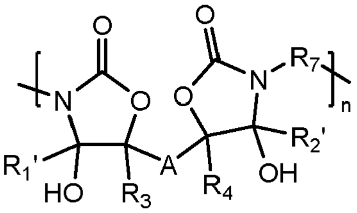
(I)
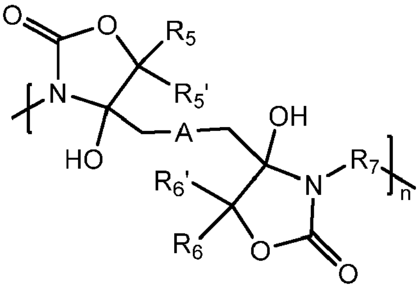
(II)

wherein

- $R_{1'}$, $R_{2'}$, independently of one another each denote a C1 to C20 alkyl group, preferably a C1 to C10 alkyl group, more preferably a C1 to C3 alkyl group, most preferably a methyl group, and
- $R_3$, $R_4$ independently of one another each denote hydrogen or a C1 to C20 alkyl group, preferably a C1 to C10 alkyl group, more preferably a C1 to C3 alkyl group, most preferably a methyl group, and
- $R_5$, $R_{5'}$, $R_6$, $R_{6'}$ independently of one another each denote hydrogen or a C1 to C20 alkyl group, preferably a C1 to C10 alkyl group, more preferably a C1 to C3 alkyl group, most preferably a methyl group, or an aryl group, preferably a phenyl group, and
- A is an organic linking group selected from a linear, branched, saturated, unsaturated, cyclic and/or non-cyclic aliphatic, aromatic or araliphatic hydrocarbon group, preferably having 1 to 20, more preferably 1 to 12, most preferably 1 to 8 carbon atoms and optionally further containing one or more functional groups selected from ether, ester, amide, amine, carboxyl, sulfur, halogen and/or urethane, and
- Optionally A, $R_3$ and $R_4$ in formula (I) are linked to each other to form a cyclic structure, and
- Optionally $R_5$ and $R_{5'}$, and/or $R_6$ and $R_{6'}$, in formula (II) are respectively linked to each other to form a cyclic structure, and
- $R_7$ is a polymeric chain selected from a polysiloxane or a polyether, and
- n is an integer from 2 to 100, preferably 2 to 50, more preferably 2 to 25, most preferably 2 to 15.

According to preferred embodiments, the poly(hydroxy oxazolidone) compounds (PHOx) is corresponding to following compound:

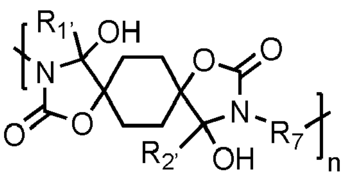

wherein

- $R_{1'}$ and $R_{2'}$ independently of one another each denote a C1 to C20 alkyl group, preferably a C1 to C10 alkyl group, more preferably a C1 to C3 alkyl group, most preferably a methyl group, and
- $R_7$ is a polymeric chain selected from a polysiloxane or a polyether, and
- n is an integer from 2 to 100, preferably 2 to 50, more preferably 2 to 25, most preferably 2 to 15.

The poly(hydroxy oxazolidone)s (PHOx) according to the invention are biocompatible and can be used at least partly in medical devices.

Advantageously, the monomeric unit of PHOx according to the invention is composed of a relatively short and rigid cyclic group with a relatively long and soft segment, originating from the polymeric chain. This provides unique properties to the PHOx.

The invention further provides formulations for forming the poly(hydroxy oxazolidone)s (PHOx), said formulation comprising at least one multifunctional cyclic carbonate having at least two terminal $\alpha$-alkylidene cyclic carbonate groups (compound A) and at least one multifunctional primary amine (compound B) wherein the multifunctional amine is composed of a polymeric chain selected from a polysiloxane and/or a polyether such as a polyalkyleneglycol having at least two primary amine groups at the end of said polymeric chain.

The at least one multifunctional cyclic carbonate is having at least two terminal $\alpha$-alkylidene cyclic carbonate groups (compound A) and is according to formula (III) or (IV):

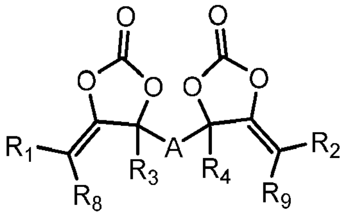 (III) 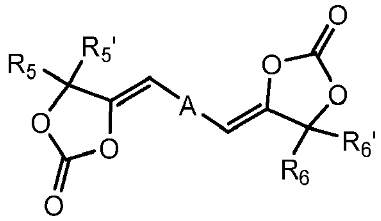 (IV)

Wherein

- $R_1$, $R_2$, $R_8$, $R_9$ independently of one another each denote hydrogen or a C1 to C20 alkyl group, preferably a C1 to C10 alkyl group, more preferably a C1 to C3 alkyl group, most preferably a hydrogen, and
- $R_3$, $R_4$, independently of one another each denote hydrogen or a C1 to C20 alkyl group, preferably a C1 to C10 alkyl group, more preferably a C1 to C3 alkyl group, most preferably a methyl group, and
- $R_5$, $R_{5'}$, $R_6$, $R_6$, independently of one another each denote hydrogen or a C1 to C20 alkyl group, preferably a C1 to C10 alkyl group, more preferably a C1 to C3 alkyl group, most preferably a methyl group, or an aryl group, preferably a phenyl group, and
- A is an organic linking group selected from a linear, branched, saturated, unsaturated, cyclic and/or non-cyclic aliphatic, aromatic or araliphatic hydrocarbon group, preferably having 1 to 20, more preferably 1 to 12, most preferably 1 to 8 carbon atoms and optionally further containing one or more functional groups selected from ether, ester, amide, amine, carboxyl, sulfur, halogen and/or urethane, and
- Optionally A, $R_3$ and $R_4$ in formula (III) are linked to each other to form a cyclic structure, and
- Optionally $R_5$ and $R_{5'}$, and/or $R_6$ and $R_{6'}$, in formula (IV) are respectively linked to each other to form a cyclic structure, and

According to preferred embodiments, the multifunctional cyclic carbonate is corresponding to following compound:

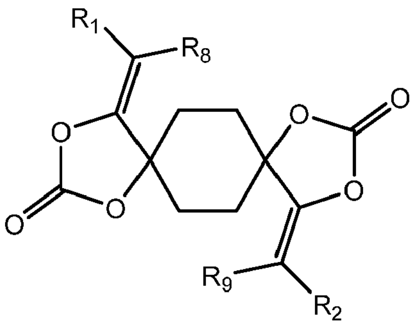

wherein

- $R_1$, $R_2$, $R_8$ and $R_9$ independently of one another each denote hydrogen or a C1 to C20 alkyl group, preferably a C1 to C10 alkyl group, more preferably a C1 to C3 alkyl group, most preferably a hydrogen.

The multifunctional amine is preferably a polymeric **diamine** containing a polymeric chain with at least two primary amine groups at the end of said polymeric chain, preferably the multifunctional amine is corresponding to the formula $NH_2$-$R_7$-$NH_2$ wherein $R_7$ is a polymeric chain selected from a polysiloxane, preferably selected from polydimethylsiloxane (PDMS) having a molecular weight in the range 900 - 30000 g/mol, preferably in the range 1500 - 10000 g/mol, more preferably in the range 2000 - 5000 g/mol, most preferably in the range 2000 - 3000 g/mol. Alternatively $R_7$ is a polymeric chain selected from a polyether, preferably a polyalkyleneglycol, more preferably a polyalkyleneglycol selected from Polytetrahydrofuran (poly(tetramethylene ether) glycol), polypropyleneglycol (PPG) or polyethylene glycol (PEG) having a molecular weight in the range 230 - 10000 g/mol, preferably 230 - 5000 g/mol, more preferably in the range 300 - 4000 g/mol, most preferably in the range 350 - 3000 g/mol.

In an alternative embodiment, the formulation of compound A and compound B recited above may be used to firstly prepare a multifunctional cyclic carbonate compound which comprises a polymeric chain coupled to at least two terminal $\alpha$-alkylidene cyclic carbonate groups, through oxazolidone bounds. The polymeric chain of the multifunctional amine is thus inserted in the multifunctional cyclic carbonate. Such polymeric compound A can then in a second step be

polymerized with a second multifunctional amine (compound C) to form an alternative PHOx in which the organic linking group A comprises a polymeric chain selected from a polysiloxane or a polyether and $R_7$ is a C1-C20 alkyl or an aryl group.

Advantageously, the formulation of compound A and compound B recited above further comprises compound C. Compound C may be selected from relatively short and/or rigid multifunctional amines, in contrast with polymeric multifunctional amines of compound B (the polymeric chain being also referred to as a long and soft segment). Compound C is for example one of the following:

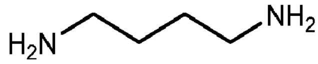

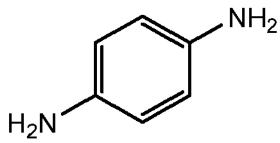

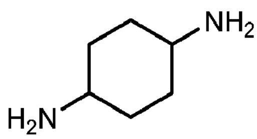

Further the invention relates to a process for forming said poly(hydroxy oxazolidone)s.

According to a second aspect, the invention relates to poly(alkylidene oxazolidone) compounds (POx), said poly(alkylidene oxazolidone) compounds preferably obtained by dehydrating the poly(hydroxy oxazolidone) compounds (PHOx) of the first aspect of the invention. The poly(alkylidene oxazolidone) compounds (POx) correspond to following formula: (V) or (VI):

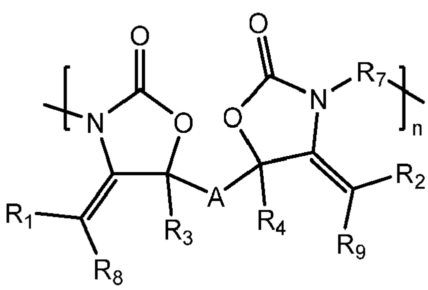
(V)

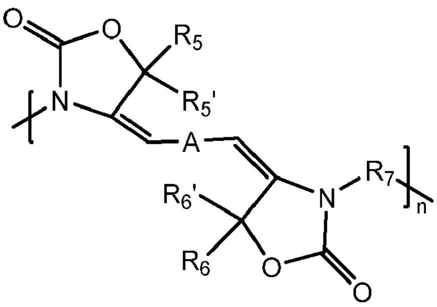
(VI)

Wherein

- $R_1$, $R_2$, $R_8$, $R_9$ independently of one another each denote hydrogen or a C1 to C20 alkyl group, preferably a C1 to C10 alkyl group, more preferably a C1 to C3 alkyl group, most preferably a hydrogen, and
- $R_3$, $R_4$, independently of one another each denote hydrogen or a C1 to C20 alkyl group, preferably a C1 to C10 alkyl group, more preferably a C1 to C3 alkyl group, most preferably a methyl group, and
- $R_5$, $R_{5'}$, $R_6$, $R_6$, independently of one another each denote hydrogen or a C1 to C20 alkyl group, preferably a C1 to C10 alkyl group, more preferably a C1 to C3 alkyl group, most preferably a methyl group, or an aryl group, preferably a phenyl group, and
- A is an organic linking group selected a linear, branched, saturated, unsaturated, cyclic and/or non-cyclic aliphatic, aromatic or araliphatic hydrocarbon group, preferably having 1 to 20, more preferably 1 to 12, most preferably 1 to 8 carbon atoms and optionally further containing one or more functional groups selected from ether, ester, amide, amine, carboxyl, sulfur, halogen and/or urethane, and
- Optionally A, $R_3$ and $R_4$ in formula (V) are linked to each other to form a cyclic structure, and
- Optionally $R_5$ and $R_{5'}$, and/or $R_6$ and $R_{6'}$, in formula (VI) are respectively linked to each other to form a cyclic structure, and
- $R_7$ is a polymeric chain selected from a polysiloxane or a polyether, and
- n is an integer from 2 to 100, preferably 2 to 50, more preferably 2 to 25, most preferably 2 to 15.

According to preferred embodiments, the poly(alkylidene oxazolidone) compounds (POx) is corresponding to following compound:

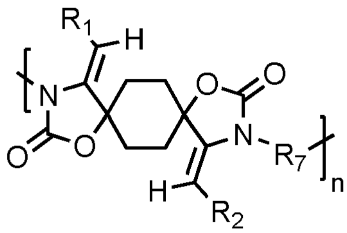

wherein

- $R_1$ and $R_2$ independently of one another each denote hydrogen or a C1 to C10 alkyl group, preferably a C1 to C6 alkyl group, more preferably a C1 to C3 alkyl group, most preferably a hydrogen, and
- $R_7$ is a polymeric chain selected from a polysiloxane or a polyether, and
- n is an integer from 2 to 100, preferably 2 to 50, more preferably 2 to 25, most preferably 2 to 15.

According to a third aspect, the current invention provides a formulation and method for cross-linking the poly(alkylidene oxazolidone) compounds according to the second aspect of the invention to form a biocompatible poly(oxazolidone) network. Said cross-linking method is preferably selected from a thiol-ene reaction wherein the poly(alkylidene oxazolidone) compounds react with polythiols and optionally a suitable radical and/or cationic polymerization initiator, more preferably a radical and/or cationic polymerization (photo-)initiator to form a cross-linked poly(oxazolidone) which surprisingly showed very good biocompatibility and extremely good mechanical resistance (especially when compared to e.g. existing PU cross-linked compounds). They also showed a surprisingly high hemocompatibility, making them particularly suitable candidates for use in any medical device in contact with blood, including but not limited to the use in a prosthetic heart valve, as medical tubing, as implants,...

DESCRIPTION OF EMBODIMENTS

For the purpose of clarity and a concise description, features are described herein as part of the same or separate embodiments, however, it will be appreciated that the scope of the invention may include embodiments having combination of all or some of the features described.

Terminology used for describing particular embodiments is not intended to limit the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise.

As used herein, the term "medical device" (also referred herein as MD) refers to all implantable foreign material for clinical use in host mammal such as for prosthetic joints, pacemakers, pacemaker leads, implantable cardioverter-defibrillators, vascular catheters, intravascular or urinary catheters, stent including coronary stent, components of extracorporeal membrane oxygenation systems or other medical tubing, blood bags and wound healing products, cardiac devices, prosthetic heart valves, chambers of left ventricular assist devices, artificial hearts, intraocular lens, dental implants, breast implants, endotracheal tubes, gastrostomy tubes and the like. Medical devices according to the invention also refer to non-implantable devices such as blood bags, wound healing products, and components of extracorporeal membrane oxygenation systems. They also include patches to manage infarcted cardiac tissue.

As used herein, the term "host mammal" refers to a human, but can also refer to an animal.

As used herein the term "thiol-ene" reaction refers to the radical and/or cationic catalyzed addition of a thiol to a vinyl functional group, thereby giving rise herein to a cross-linked oxazolidone polymer network, also referred to as cross-linked poly(oxazolidone) network, cross-linked poly(oxazolidone) compound or cross-linked poly(oxazolidone). The "thiol-ene" reaction may be enhanced by the presence of a radical and or cationic polymerization initiator such as a photo-initiator or a conventional radical and/or cationic initiator.

As used herein, the term "wt %", or "weight percentage", or "percentage by weight", refers to the mass fraction of a compound in a mixture divided by the total mass of said mixture, expressed as a percentage between 0 and 100.

According to a first aspect of the invention, a poly(hydroxy oxazolidone) compound (PHOx) is provided, said

compound corresponding to formula (I) or (II)

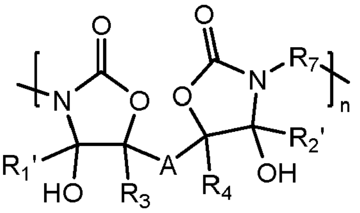
(I)

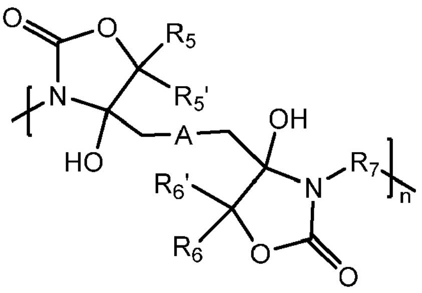
(II)

wherein

- $R_{1'}$, $R_{2'}$ independently of one another each denote a C1 to C20 alkyl group, preferably a C1 to C10 alkyl group, more preferably a C1 to C3 alkyl group, most preferably a methyl group, and
- $R_3$, $R_4$ independently of one another each denote hydrogen or a C1 to C20 alkyl group, preferably a C1 to C10 alkyl group, more preferably a C1 to C3 alkyl group, most preferably a methyl group, and
- $R_5$, $R_{5'}$, $R_6$, $R_{6'}$ independently of one another each denote hydrogen or a C1 to C20 alkyl group, preferably a C1 to C10 alkyl group, more preferably a C1 to C3 alkyl group, most preferably a methyl group, or an aryl group, preferably a phenyl group, and
- A is an organic linking group preferably selected from a linear, branched, saturated, unsaturated, cyclic and/or non-cyclic aliphatic, aromatic or araliphatic hydrocarbon group, preferably having 1 to 20, more preferably 1 to 12, most preferably 1 to 8 carbon atoms and optionally further containing one or more functional groups selected from ether, ester, amide, amine, carboxyl, sulfur, halogen and/or urethane, and
- Optionally A, $R_3$ and $R_4$ in formula (I) are linked to each other to form a cyclic structure, and
- Optionally $R_5$ and $R_{5'}$, and/or $R_6$ and $R_{6'}$, in formula (II) are respectively linked to each other to form a cyclic structure, and
- $R_7$ is a polymeric chain selected from a polysiloxane or a polyether, and
- n is an integer from 2 to 100, preferably 2 to 50, more preferably 2 to 25, most preferably 2 to 15.

According to preferred embodiments, the poly(hydroxy oxazolidone) compound (PHOx) is corresponding to following compound:

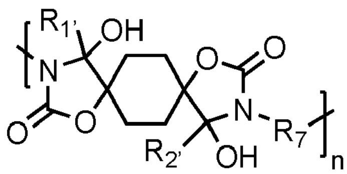

wherein

- $R_{1'}$ and $R_{2'}$ independently of one another each denote a C1 to C20 alkyl group, preferably a C1 to C10 alkyl group, more preferably a C1 to C3 alkyl group, most preferably a methyl group, and
- $R_7$ is a polymeric chain selected from a polysiloxane or a polyether, and
- n is an integer from 2 to 100, preferably 2 to 50, more preferably 2 to 25, most preferably 2 to 15.

According to embodiments, the polymeric chain is selected from a polysiloxane, preferably polydimethylsiloxane (PDMS). Polysiloxane may have a molecular weight in the range 250 - 50 000 g/mol, preferably 900 - 30 000 g/mol, more preferably 1500 - 10 000 g/mol, more preferably in the range 2000 - 5000 g/mol, most preferably in the range 2000 - 3000 g/mol.

According to embodiments, the polymeric chain is selected from a polyether selected from a polyalkylene glycol, preferably Polytetrahydrofuran (poly(tetramethylene ether) glycol), polypropylene glycol (PPG) or polyethylene glycol (PEG). The polyether may have a molecular weight in the range 230 - 50 000 g/mol, preferably in the range 230 - 10 000 g/mol, more preferably in the range 230 - 5000 g/mol, more preferably 300 - 4000 g/mol, most preferably in the range 350 - 3000 g/mol.

The invention further provides a formulation for preparing a poly(hydroxy oxazolidone) such as for example the

poly(hydroxy oxazolidone) according to formula (I) or (II), said formulation comprising:

- at least one multifunctional cyclic carbonate having at least two terminal α-alkylidene cyclic carbonate groups (compound A) according to formula (III) or (IV)

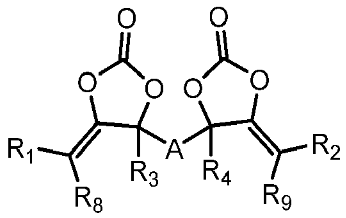
(III)
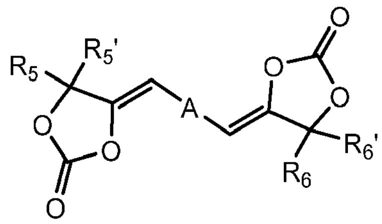
(IV)

Wherein

- $R_1$, $R_2$, $R_8$, $R_9$ independently of one another each denote hydrogen or a C1 to C20 alkyl group, preferably a C1 to C10 alkyl group, more preferably a C1 to C3 alkyl group, most preferably a hydrogen, and
- $R_3$, $R_4$ independently of one another each denote hydrogen or a C1 to C20 alkyl group, preferably a C1 to C10 alkyl group, more preferably a C1 to C3 alkyl group, most preferably a methyl group, and
- $R_5$, $R_{5'}$, $R_6$, $R_6$, independently of one another each denote hydrogen or a C1 to C20 alkyl group, preferably a C1 to C10 alkyl group, more preferably a C1 to C3 alkyl group, most preferably a methyl group, or an aryl group, preferably a phenyl group, and
- A is an organic linking group preferably selected from a linear, branched, saturated, unsaturated, cyclic and/or non-cyclic aliphatic, aromatic or araliphatic hydrocarbon group, preferably having 1 to 20, more preferably 1 to 12, most preferably 1 to 8 carbon atoms and optionally further containing one or more functional groups selected from ether, ester, amide, amine, carboxyl, sulfur, halogen and/or urethane.
- Optionally A, $R_3$ and $R_4$ in formula (III) are linked to each other to form a cyclic structure
- Optionally $R_5$ and $R_{5'}$, and/or $R_6$ and $R_{6'}$, in formula (IV) are respectively linked to each other to form a cyclic structure, and

- at least one multifunctional amine (compound B) wherein the multifunctional **amine** is composed of a polymeric chain having at least two primary amine groups at the end of said polymeric chain and wherein the polymer chain is selected from a polysiloxane or a polyether such as a polyalkyleneglycol.

According to preferred embodiments, the multifunctional cyclic carbonate is corresponding to following compound:

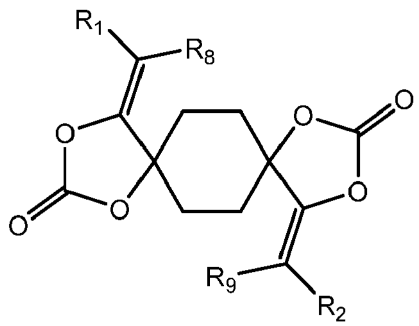

wherein

- $R_1$, $R_2$, $R_8$ and $R_9$ independently of one another each denote hydrogen or a C1 to C20 alkyl group, preferably a C1 to C10 alkyl group, more preferably a C1 to C3 alkyl group, most preferably a hydrogen atom.

According to preferred embodiments, the multifunctional amine is a polymeric diamine containing a polymeric chain with at least two primary amine groups at the end of said polymeric chain, preferably the multifunctional amine is corresponding to the formula $NH_2$-$R_7$-$NH_2$ wherein $R_7$ is composed of a polymeric chain selected from a polysiloxane or a polyether such as a polyalkyleneglycol.

According to preferred embodiments, the multifunctional amine is corresponding to the formula $NH_2$-$R_7$-$NH_2$ wherein $R_7$ is selected from polydimethylsiloxane (PDMS) having a molecular weight in the range 900 - 30000 g/mol, preferably in the range 1500 - 10000 g/mol, more preferably in the range 2000 - 5000 g/mol, most preferably in the range 2000 - 3000 g/mol.

According to preferred embodiments, the multifunctional amine is corresponding to the formula $NH_2$-$R_7$-$NH_2$ wherein $R_7$ is selected from polypropylene glycol (PPG) or polyethylene glycol (PEG) having a molecular weight in the range 230 - 10000 g/mol, preferably in the range 230 - 5000 g/mol, more preferably in the range 300 - 4000 g/mol, most preferably in the range 350 - 3000 g/mol.

The invention further provides a process for forming poly(hydroxy oxazolidone) compounds, such as poly(hydroxy oxazolidone) compounds according to formula (I) or (II), said process comprising at least a polyaddition step between the multifunctional cyclic carbonate (compound A) and the multifunctional amine (compound B).

**Multifunctional cyclic carbonates (compound A)**

The multifunctional cyclic carbonates according to the invention used to form the poly(hydroxy oxazolidone)s of the present invention have at least two terminal $\alpha$-alkylidene cyclic carbonate groups and are also referred to herein as $\alpha$-alkylidene bis(cyclic carbonate)s.

The multifunctional cyclic carbonates according to the invention correspond to formula (III) or (IV)

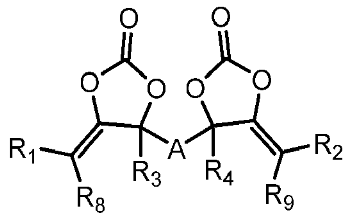
(III)
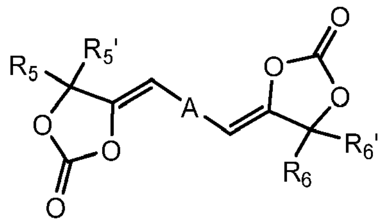
(IV)

Wherein

- $R_1$, $R_2$, $R_8$, $R_9$ independently of one another each denote hydrogen or a C1 to C20 alkyl group, preferably a C1 to C10 alkyl group, more preferably a C1 to C3 alkyl group, most preferably a hydrogen, and
- $R_3$, $R_4$ independently of one another each denote hydrogen or a C1 to C20 alkyl group, preferably a C1 to C10 alkyl group, more preferably a C1 to C3 alkyl group, most preferably a methyl group, and
- $R_5$, $R_{5'}$, $R_6$, $R_6$, independently of one another each denote hydrogen or a C1 to C20 alkyl group, preferably a C1 to C10 alkyl group, more preferably a C1 to C3 alkyl group, most preferably a methyl group, or an aryl group, preferably a phenyl group, and
- A is an organic linking group preferably selected from a linear, branched, saturated, unsaturated, cyclic and/or non-cyclic aliphatic, aromatic or araliphatic hydrocarbon group, preferably having 1 to 20, more preferably 1 to 12, most preferably 1 to 8 carbon atoms and optionally further containing one or more functional groups selected from ether, ester, amide, amine, carboxyl, sulfur, halogen and/or urethane
- Optionally A, $R_3$ and $R_4$ in formula (III) are linked to each other to form a cyclic structure.

According to preferred embodiments, $R_1$ and $R_2$ independently of one another preferably may be selected from a C1 to C10 alkyl group, a C1 to C6 alkyl group, a C1 to C3 alkyl group, a methyl group, or particularly preferably an H atom.

According to preferred embodiments, the multifunctional cyclic carbonate is corresponding to following compound:

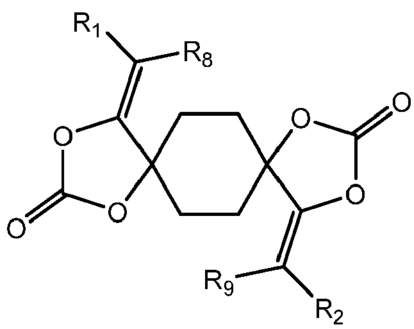

wherein

- $R_1$, $R_2$, $R_8$ and $R_9$ independently of one another each denote hydrogen or a C1 to C20 alkyl group, preferably a C1 to C10 alkyl group, more preferably a C1 to C3 alkyl group, most preferably a hydrogen atom.

According to preferred embodiments, the multifunctional cyclic carbonates according to the invention correspond to formula (III)

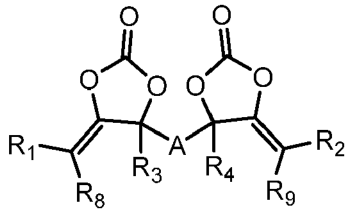
(III)

Wherein $R_1$, $R_2$, $R_8$, $R_9$ independently of one another are preferably selected from a C1 to C10 alkyl group, a C1 to C6 alkyl group, a C1 to C3 alkyl group, a methyl group, or particularly preferably an H atom and wherein R3 and R4 independently of one another preferably may be selected from a C1 to C10 alkyl group, a C1 to C6 alkyl group, a C1 to C3 alkyl group or particularly preferably a hydrogen.

According to preferred embodiments, the multifunctional cyclic carbonates according to the invention correspond to formula (IV)

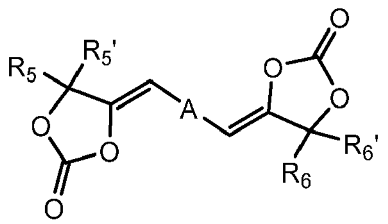
(IV)

Wherein $R_{5'}$, $R_5$, $R_6$' and $R_6$ independently of one another are preferably selected from a C1 to C10 alkyl group, a C1 to C6 alkyl group, a C1 to C3 alkyl group, a methyl group, or particularly preferably a methyl group.

According to preferred embodiments, $R_3$ and $R_4$ preferably stands for a C1 to 10 alkyl group, a C1 to C6 alkyl group, a C1 to C3 alkyl group, or for an H atom, particularly preferably for a methyl group. $R_1$, $R_2$, $R_8$, $R_9$ particularly preferably stand for hydrogen or $R_1$, $R_2$, $R_8$ and $R_9$ stand for hydrogen and $R_3$ stands for a methyl group.

According to preferred embodiments, $R_5$, $R_6$, $R_5$, and $R_6$ preferably have the same meaning.

According to preferred embodiments, $R_1$ preferably has the same meaning as $R_2$, $R_8$ has the same meaning as $R_9$ and $R_4$ preferably has the same meaning as $R_3$.

According to preferred embodiments, A is an organic linking group, preferably selected from an alkylene group preferably having 1 to 6 carbon atoms, e.g., butylene, propylene, ethylene, or particularly preferably methylene and may further contain one or more functional groups selected from ether, ester, amide, amine, carboxyl, sulfur, halogen and/or urethane.

Suitable examples of $\alpha$-alkylidene bis(cyclic carbonate)s correspond to formula III and are corresponding to following compounds (also referred herein as BisaCC1 (left) and BisaCC2 (right)):

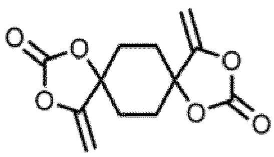

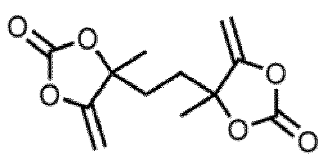

Further suitable examples of $\alpha$-alkylidene bis(cyclic carbonate)s corresponding to formula III are following compounds:

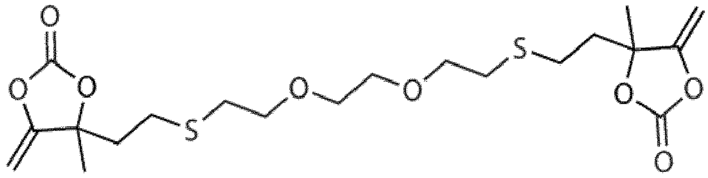

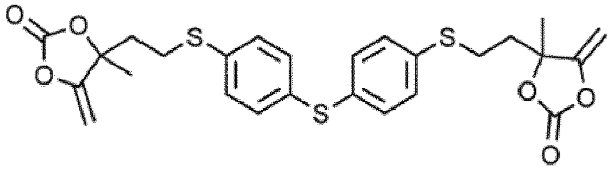

Suitable examples of $\alpha$-alkylidene bis(cyclic carbonate)s correspond to formula IV and are corresponding to following compound:

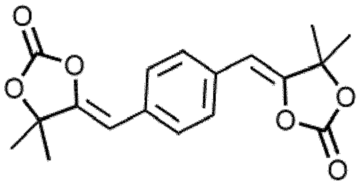

**Polymeric diamines (compound B)**

The polymeric amines according to the invention used to form the poly(hydroxy oxazolidone)s of the present invention are preferably selected from polymeric diamines wherein the diamine is containing a polymeric chain having at least two primary amine groups at the end of said polymeric chain.

The polymeric diamine according to the invention is corresponding to the formula $NH_2$-$R_7$-$NH_2$ wherein $R_7$ is composed of a polymeric chain selected from a polysiloxane or a polyether such as a polyalkyleneglycol.

Preferred polymeric diamines are selected from polymeric diamines comprising a polymeric chain selected from polydimethylsiloxane (PDMS), polypropylene glycol (PPG) or polyethylene glycol (PEG) having at least two primary amine groups at the end of said polymeric chain.

According to preferred embodiments, the polymeric chain is selected from a polysiloxane, preferably PDMS having a molecular weight in the range 1500 - 5000 g/mol, more preferably in the range 2000 - 4000 g/mol, most preferably in the range 2000 - 3000 g/mol.

According to embodiments, the multifunctional amine is composed of a polymeric chain selected from a polyether, preferably a polyalkylene glycol, more preferably selected from PEG having a molecular weight in the range 230 - 5000 g/mol, more preferably in the range 300 - 4000 g/mol, most preferably in the range 350 - 3000 g/mol.

According to embodiments, the multifunctional amine is composed of a polymeric chain selected from a polyether, preferably a polyalkylene glycol, more preferably selected from PPG having a molecular weight in the range 230 - 5000 g/mol, more preferably in the range 300 - 4000 g/mol, most preferably in the range 350 - 3000 g/mol.

Examples of suitable polymeric diamines containing a polymeric chain with at least two primary amine groups at the end of said polymeric chain are commercially available Jeffamine® ED series, Jeffamine® D series, Jeffamine® M series available from Huntsman.

The use of polymeric diamines wherein the polymeric chain is selected from PDMS lead to poly(hydroxy oxazolidone)s having an elastomeric character.

The use of polymeric diamines wherein the polymeric chain is selected from PPG or PEG lead to poly(hydroxy oxazolidone)s being more hydrophilic compared to poly(hydroxy oxazolidone)s made using PDMS. The use of PDMS in the formulation for making the poly(hydroxy oxazolidone)s will lead to poly(hydroxy oxazolidone)s having more hydrophobicity.

Suitable examples of PPG and PEG based polymeric diamines (also referred to herein as PPG-$NH_2$ and PEG-$NH_2$) according to the invention are:

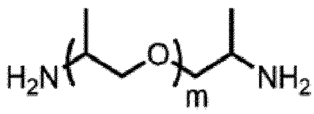 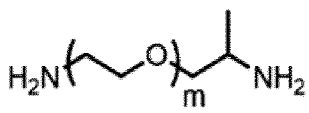

wherein m is an integer in the range 3 to 1000, preferably 3 to 300, more preferably 3 to 112.

Suitable examples of PDMS based polymeric diamines (also referred to herein as PDMS-$NH_2$) according to the invention are:

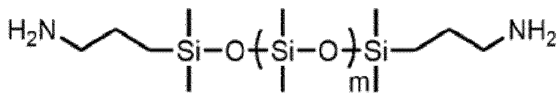

wherein m is an integer in the range 3 to 1000, preferably 6 to 500, more preferably 8 to 362.

Specific examples of suitable diamines are

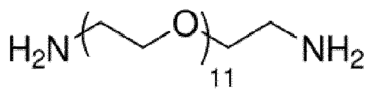 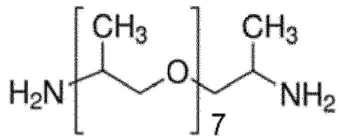

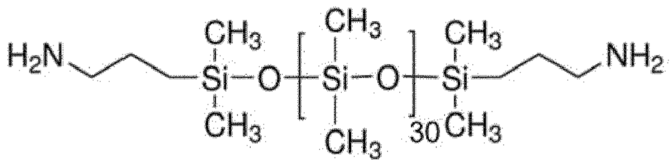

**Polyaddition step (between compound A and compound B)**

An example of a polyaddition reaction according to the invention is as follows:

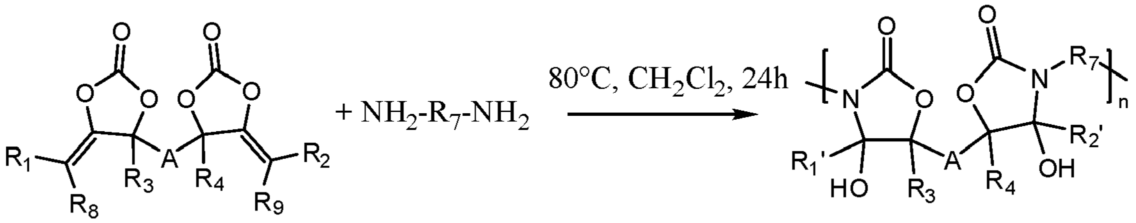


Some possible examples of polyhydroxy oxazolidones according to the present invention are as follows:

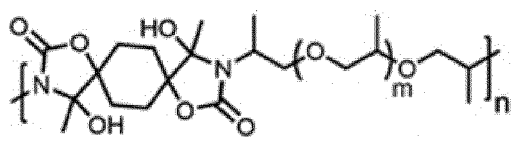

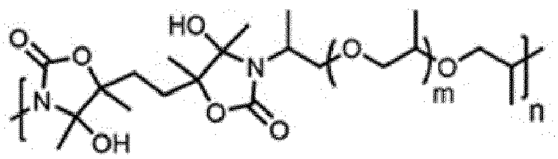

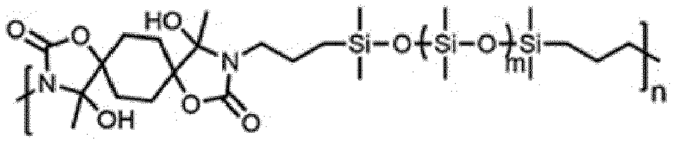

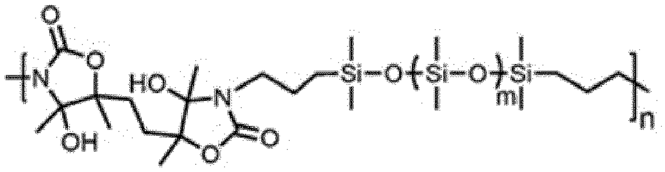

Wherein m is 3 - 112 and n is 8 - 362.

According to preferred embodiments, the polyaddition step is performed by reacting the multifunctional cyclic carbonate (compound A) with the multifunctional amine (compound B) by mixing them at a temperature in the range 25 to 100 °C, preferably 60 to 90°C optionally in the presence of an organic solvent and preferably under nitrogen atmosphere. In case a solvent is used, the solvent may be selected from organic solvents such as chloroform or dichloromethane.

According to a second aspect of the invention, a poly(alkylidene oxazolidone) compound is provided, said compound corresponding to formula (V) or (VI):

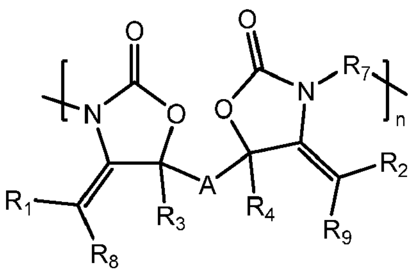

(V)

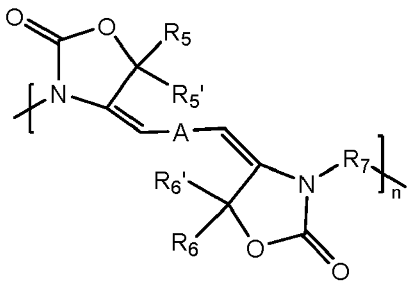

(VI)

wherein

- $R_1$, $R_2$, $R_8$, $R_9$ independently of one another each denote hydrogen or a C1 to C20 alkyl group, preferably a C1 to C10 alkyl group, more preferably a C1 to C3 alkyl group, most preferably a hydrogen, and
- $R_3$, $R_4$ independently of one another each denote hydrogen or a C1 to C20 alkyl group, preferably a C1 to C10 alkyl group, more preferably a C1 to C3 alkyl group, most preferably a methyl group, and
- $R_5$, $R_{5'}$, $R_6$, $R_{6'}$ independently of one another each denote hydrogen or a C1 to C20 alkyl group, preferably a C1 to C10 alkyl group, more preferably a C1 to C3 alkyl group, most preferably a methyl group, or an aryl group, preferably a phenyl group, and
- A is an organic linking group selected a linear, branched, saturated, unsaturated, cyclic and/or non-cyclic aliphatic, aromatic or araliphatic hydrocarbon group, preferably having 1 to 20, more preferably 1 to 12, most preferably 1 to 8 carbon atoms and optionally further containing one or more functional groups selected from ether, ester, amide, amine, carboxyl, sulfur, halogen and/or urethane, and
- Optionally A, $R_3$ and $R_4$ in formula (V) are linked to each other to form a cyclic structure, and
- Optionally $R_5$ and $R_{5'}$, and/or $R_6$ and $R_{6'}$, in formula (VI) are respectively linked to each other to form a cyclic structure, and
- $R_7$ is a polymeric chain selected from a polysiloxane or a polyether, and
- n is an integer from 2 to 100, preferably 2 to 50, more preferably 2 to 25, most preferably 2 to 15.

According to preferred embodiments, the poly(alkylidene oxazolidone) compound according to the invention is corresponding to formula (V):

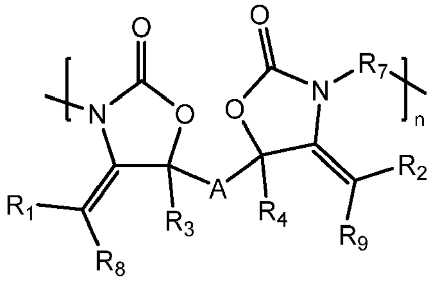

(V)

wherein

- $R_1$, $R_2$, $R_8$ and $R_9$ independently of one another preferably may be selected from a hydrogen or a C1 to C10 alkyl group, a C1 to C6 alkyl group, a C1 to C3 alkyl group, a methyl group, particularly preferably $R_1$ and/or $R_2$ and/or $R_8$ and/or $R_9$ are selected from an H atom.
- $R_3$ and $R_4$ may be selected from a hydrogen or a C1 to 10 alkyl group, a C1 to C6 alkyl group, a C1 to C3 alkyl group, particularly preferably a methyl group.
- $R_1$, $R_2$, $R_8$, $R_9$ particularly preferably stand for hydrogen or $R_1$, $R_2$, $R_8$ and $R_9$, stand for hydrogen and $R_3$, $R_4$ stands for a methyl group,
- A is an organic linking group, preferably selected from a hydrocarbon group, in particular an alkylene group preferably having 1 to 6 carbon atoms, e.g., butylene, propylene, ethylene, or particularly preferably methylene and A may further contain one or more functional groups selected from functional groups selected from ether, ester, amide, amine, carboxyl, sulfur, halogen and/or urethane, and
- Optionally A, $R_3$ and $R_4$ in formula (V) are linked to each other to form a cyclic structure, and
- $R_7$ is a polymeric chain selected from a polysiloxane or polyether, and
- n is an integer from 2 to 100, preferably 2 to 50, more preferably 2 to 25, most preferably 2 to 15.

According to preferred embodiments, the poly(alkylidene oxazolidone) compound according to the invention is corresponding to formula (VI):

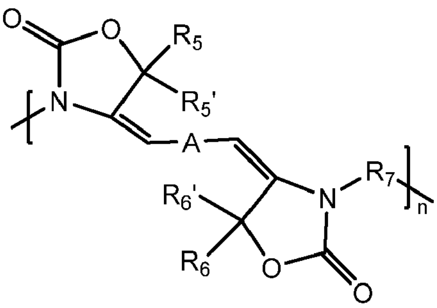

(VI)

Wherein

- $R_5$, $R_6$, $R_{5'}$, and $R_{6'}$, may be selected from a C1 to 10 alkyl group, a C1 to C6 alkyl group, a C1 to C3 alkyl group, or for an H atom, particularly preferably for a methyl group, or may be selected from an aryl group, preferably a phenyl group, and
- A is an organic linking group, preferably selected from a divalent hydrocarbon group, in particular an alkylene group preferably having 1 to 6 carbon atoms, e.g. butylene, propylene, ethylene, or particularly preferably methylene or an aromatic group and A may further contain one or more functional groups selected from functional groups selected from ether, ester, amide, amine, carboxyl, sulfur, halogen and/or urethane, and
- Optionally $R_5$ and $R_{5'}$, and/or $R_6$ and $R_{6'}$, in formula (VI) are respectively linked to each other to form a cyclic structure, and
- $R_7$ is a polymeric chain selected from a polysiloxane or polyether, and
- n is an integer from 2 to 100, preferably 2 to 50, more preferably 2 to 25, most preferably 2 to 15.

The invention further provides a process for forming the poly(alkylidene oxazolidone) compounds corresponding to formula (V) or (VI), said process comprising at least the step of dehydrating poly(hydroxy oxazolidone) compounds such

as the poly(hydroxy oxazolidone) compounds according to the first aspect of the invention.

**Dehydrating step**

The dehydration step may be performed in an oven, optionally under vacuum conditions, at a temperature in the range 100°C up to 200°C , preferably in the range 100°C up to 160°C, such as 130°C for at least 1h, most preferably 2 h under preferably solvent- and catalyst-free conditions.

An example of a dehydration reaction according to the invention is as follows:

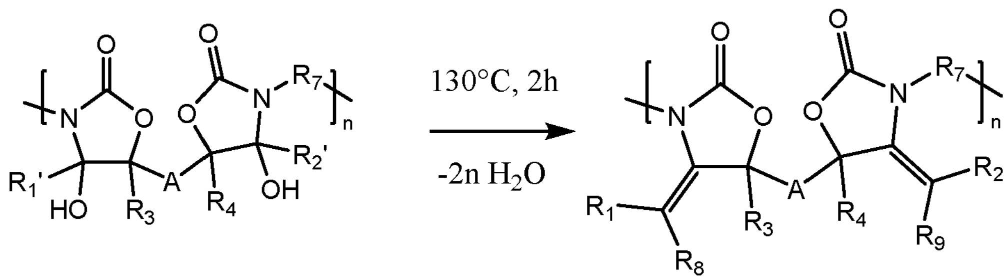


Alternatively, the dehydration may be performed by reflux wherein the polymer is dispersed in glacial acetic acid for 2h after which the acetic acid is removed from the polymer. Alternative acids may be used as well such as methane sulfonic acid and/or sulfuric acid) to dehydrate the polymers after having the polymers being solubilized in an organic solvent (such as DMF, DMSO).

According to a third aspect, the current invention provides a formulation and method for cross-linking the poly(alkylidene oxazolidone) compounds according to the second aspect of the invention to form a biocompatible poly(oxazolidone) network. Said cross-linking method is preferably selected from a thiol-ene reaction wherein the poly(alkylidene oxazolidone) compounds react with polythiol and optionally a suitable radical/cationic polymerization initiator, preferably selected from a radical/cationic polymerization photo-initiator or radical/cationic polymerization thermal initiator to form a cross-linked poly(oxazolidone) which surprisingly showed biocompatibility and extremely good mechanical resistance, comparable to PU cross-linked compounds. They also showed a surprisingly high hemocompatibility, making them particularly suitable candidates for use in any medical device in contact with blood, including but not limited to the use in a prosthetic heart valve, as medical tubing, as implants,...

According to embodiments, the cross-linkable formulation for forming a network of poly(oxazolidone) compounds is comprising:

- At least one poly(alkylidene oxazolidone) compound according to second aspect of the invention, and
- At least one polythiol corresponding to formula $R_8$-$SH_x$ wherein $R_8$ is an organic group selected from a C4-C20 alkyl group, and optionally further containing one or more functional groups selected from ether, ester, amide, amine, carboxyl, sulfur, halogen and/or urethane and x is an integer between 2-8, preferably between 2 and 6, and
- Optionally a radical polymerization initiator, preferably selected from a radical photopolymerization initiator and/or a radical thermal polymerization initiator and/or redox radical initiators, and
- Optionally a cationic polymerization initiator, preferably selected from a cationic photopolymerization initiator and/or a thermal cationic polymerization initiator.
- Optionally a combination of radical and cationic initiators.

The invention provides a cross-linking reaction for cross-linkable formulation for forming a network of poly(oxazolidone) compounds, said cross-linking step is preferably selected from a radical and/or cationic polymerization, more in particular a thiol-ene reaction wherein the poly(alkylidene oxazolidone) compounds react with polythiols and optionally a suitable radical and/or cationic polymerization initiator.

Cross-linked poly(oxazolidone) compounds according to the invention being cross-linked by means of cationic polymerization using a cationic polymerization initiator tend to be more flexible and suitable for use in medical application requiring flexibility such as heart valves.

Cross-linked poly(oxazolidone) compounds according to the invention being cross-linked by means of cationic polymerization using a cationic polymerization initiator are recyclable due to the presence of dynamic bonds.

The invention also provides recyclable cross-linked poly(oxazolidone)s obtained by the process described above and in the presence of a cationic polymerization initiator, preferably selected from acids such as methane sulfonic acid, sulfuric acid, p-toluene sulfonic acid and combinations thereof.

The invention also provides a process for recycling the cross-linked poly(oxazolidone)s described above,

preferably by means of injection molding, extrusion and extrusion-based additive manufacturing, or chemical recycling.

Preferably said thiol-ene reaction is performed at room temperature and with irradiation with light (selected from UV, red and/or visible light range) and the initiator is selected from a photo-polymerization initiator.

Cross-linking as described herein may also be referred to as curing or reticulation. By cross-linkable formulation is also meant a curable formulation.

The cross-linked poly(oxazolidone) compounds are ideally suited for use in medical devices seen the biocompatibility.

**Polythiols**

Suitable polythiols for use in the cross-linkable formulation for making cross-linked poly(oxazolidone) networks refers to compounds containing two or more thiol functional groups (-SH).

The polythiols (b) for use in the present invention include materials of the formula $R_8$-$SH_x$ wherein $R_8$ is an organic moiety and x is an integer of at least 2, typically 2 to 6. Such polythiols may for instance comprise a reaction product of a thiol-functional organic acid and a polyol. Accordingly, the organic moiety $R_8$ can contain ester groups and/or be derived from a polyol.

Examples of suitable polythiols that can be used in the cross-linkable compositions according to the present invention may thus e.g. include esters of thiol-containing acids.

Examples of suitable polythiols:

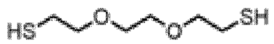

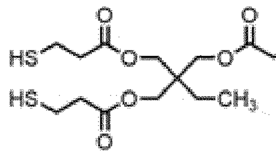

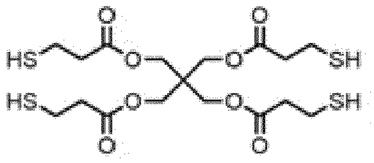

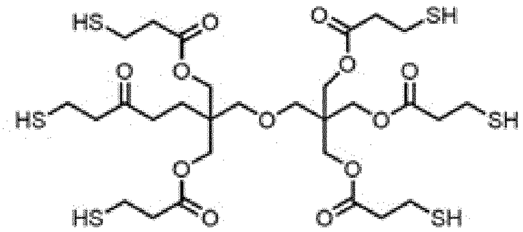

**Initiators for cross-linking**

The cross-linkable formulation of the present invention used to form the cross-linked (bio-compatible) poly(alkylidene oxazolidone)s of the present invention may contain a radical or cationic polymerization initiator in the thiol-ene reaction.

According to embodiments, the radical polymerization initiator refers to one that generates a radical due to heat, (UV) light, a redox reaction, or the like. As such, the initiator may be selected from a photopolymerization initiator and/or a thermal polymerization initiator.

Examples of suitable radical thermal polymerization initiator include organic peroxides, azo compounds, redox initiators, and the like.

Examples of the organic peroxides include benzoyl peroxide, cumene hydroperoxide, di-tert-butyl peroxide, tert-butyl hydroperoxide and dicumyl peroxide.

Specific examples of the azo compound include 2,2'-azobispropane, 2,2'-dichloro-2,2'-azobispropane, 1,1'-azo(methylethyl) diacetate, 2,2'-azobisisobutane, 2,2'-azobisisobutylamide, 2,2'-azobisisobutyronitrile (AIBN), 2,2'-azobis-2-methyl methylpropionate, 2,2'-dichloro-2,2'-azobisbutane, 2,2'-azobis-2-methylbutyronitrile, 2,2'-dimethyl azobisisobutyrate, 3,5-dihydroxymethylphenylazo-2-methylmalonodinitrile, 2,2'-azobis-2-methylvaleronitrile, 4,4'-azobis-4-dimethyl cyanovaleric acid, and 2,2'-azobis-2,4-dimethylvaleronitrile.

Examples of suitable redox initiators include, for example, combinations of hydrogen peroxide-iron (II) salt, organic peroxide-dimethylaniline, cerium (IV) salt-alcohol, and the like can be mentioned.

Examples of suitable radical photopolymerization initiators include known photopolymerization initiators such as

alkylphenone-based photopolymerization initiators, α-aminoalkyl ketone-based photopolymerization initiators, and phosphine oxide-based photopolymerization initiators.

More specifically, the radical photopolymerization initiator may be selected from a alkylphenone-based photopolymerization initiators such as 1-hydroxycyclohexyl phenyl ketone, 2,2-dimethoxy-1,2-diphenylethan-1-one, 2-hydroxy-2-methyl-1-phenylpropan-1-one, 1-[4-(2-hydroxyethoxy)-phenyl]-2-hydroxy-2-methyl-1-propan-1-one, 2-hydroxy-1-{4-[4-(2-hydroxy-2-methylpropionyl)benzyl]phenyl}-2-methylpropan-1-one, and the like can be mentioned.

Examples of suitable α-aminoalkyl ketone-based photopolymerization initiators include 2-methyl-1-[4-(methylthio)phenyl]-2-morpholinopropan-1-one, 2-benzyl-2-dimethylamino-1-(4-morpholinophenyl)-1-butanone, 2-dimethylamino-2-(4-methylbenzyl)-1-(4-morpholin-4-yl-phenyl) butan-1-one, and the like.

Examples of suitable phosphine oxide-based photopolymerization initiators include bis(2,4,6-trimethylbenzoyl) phenyl phosphine oxide, bis(2,6-dimethoxybenzoyl)-2,4,4-trimethylpentyl phosphine oxide, 2,4,6-trimethylbenzoyl-diphenyl phosphine oxide, and the like. In addition, two or more types of these photopolymerization initiators may be added as a mixture.

As other suitable polymerization initiators, dialkyl disulfides may be used such as tetraalkyl thiuram disulfide in which the S-S bonds are easily decomposed by heat.

According to embodiments, the cationic polymerization initiator may be selected from acids such as methane sulfonic acid, sulfuric acid, p-toluene sulfonic acid and combinations thereof. Cationic photoinitiators can also be used.

The amount of the cationic / radical polymerization initiator used is preferably in the range from 0.001 to 10 wt%, preferably from 0.005 to 5 wt% more preferably from 0.01 to 3 wt% calculated on the total weight of the cross-linkable poly(alkylidene oxazolidone) formulation.

**Use of the poly(oxazolidone) derivatives in medical devices**

The poly(oxazolidone) compounds selected from poly(hydroxyoxazolidone)s according to the first aspect of the invention and the cross-linked poly(oxazolidone) compounds according to the third aspect of the invention can be used as main component of a medical device's bulk composition or as a coating at the surface thereof. The poly(alkylidene oxazolidone) compounds of the second aspect of the invention are also suitable in a process for making a medical device when used in a cross-linkable composition thereby resulting in biocompatible cross-linked poly(oxazolidone) compounds of the third aspect of the invention.

All poly(oxazolidone) derivatives according to the invention are hemocompatible, more in particular the PHOx compounds according to the invention are advantageously more hemocompatible compared to state of the art isocyanate-based polyurethanes. They induce less coagulation and less platelet adhesion than isocyanate-based polyurethanes. The materials further advantageously induce less bacteria adhesion than conventional materials used for manufacturing catheters. In addition, the materials have a high mechanical resistance, present a high mechanical strength, an adaptable elasticity and surprisingly a high resistance in aqueous environments. These properties make them ideally suitable for use as medical devices when they are in contact with blood (or other fluid outside the body).

Since thrombosis of medical devices significantly increases the risk of bacterial infection, the invention will also reduce infection, the second major complication of medical device usage.

Medical devices also refer to non-implantable devices such as blood bags, wound healing products, and components of extracorporeal membrane oxygenation systems. They also include patches to manage infarcted cardiac tissue.

Particularly the medical device comprising the poly(oxazolidone) derivatives according to the first and third aspect of the invention is a catheter that is introduced in the host mammal to deliver fluids such as blood products, glucose solutions, medications diagnostic agents and so forth. Catheters may also be used for example to withdrawing blood from vasculature in order to treat blood or to drain urine from urinary bladder or for gathering of samples.

The catheter according to the invention refers to urinary catheters, central venous catheters, cardiovascular catheters, neurovascular catheters, intravenous catheters, and specialty catheters, but also endotracheal tubes, cannulae, ophthalmic catheter and the like. The catheter according to the invention may be partially or totally made of the poly(oxazolidone) derivatives according to the first and third aspect of the invention.

Alternatively, the catheter according to the invention may be partially or totally coated with the poly(oxazolidone) derivative.

Particularly the medical device comprising the poly(oxazolidone) derivative is a prosthetic heart valve such as a polymeric valve, a mechanical valve or a bioprosthetic valve.

A prosthetic heart valve comprising poly(oxazolidone) derivative may be partially or totally made of poly(oxazolidone) derivative.

Alternatively, the prosthetic heart valve comprising poly(oxazolidone) derivative may be partially or totally coated with poly(oxazolidone) derivative.

A mechanical valve refers to mono or bi-leaflet heart valve made of either pyrolytic carbon or titanium coated with

pyrolytic carbon and further coated with poly(oxazolidone) derivative.

A bioprosthetic valve also called tissue valve refers to bi-leaflet or tri-leaflet heart valve having tissues components provided by a donor animal and further coated with poly(oxazolidone) derivative.

The polymeric valve refers to bi-leaflet or tri-leaflet heart valve totally or partly made from poly(oxazolidone) derivative. The polymeric valve may also be totally or partly coated on its surface with poly(hydroxyurethane) (PHU), when made from another polymeric composition.

The new medical device comprising said poly(oxazolidone) derivative according to the present invention, either as a coating at the surface thereof or as main component of the medical device's bulk composition, is advantageously more hemocompatible, inducing less coagulation and less platelet adhesion than medical device comprising isocyanate-based polyurethane.

**Process for preparing medical device**

The poly(oxazolidone) derivatives according to the invention may advantageously be processed into medical device by a wide variety of processing techniques.

When the poly(alkylidene oxazolidone) derivatives of the invention are in the form of a thermoplastic resin, said resin can be molded and/or extruded and/or 3D printed. For example by means of injection molding to obtain a cross-linked poly(oxazolidone) comprising medical device. Extrusion-based additive manufacturing/3D printing techniques may be selected from fused deposition modeling/ fused filament fabrication and direct ink writing.

Alternatively, the cross-linkable formulation comprising the poly(alkylidene oxazolidone) derivatives of the invention poly(alkylidene oxazolidone) derivatives of the invention are in the form of a liquid. A thiol and optionally a suitable radical and/or cationic initiator may be added to form a curable liquid resin. In case of a photoinitiator, it may be processed (cross-linked) by light-based 3D printing techniques, such as stereolithography. The liquid resin may be cured by UV light and/or visible light. It may be processed by digital laser printing.

The photo-curable liquid resin may also be irradiated in a mold.

Advantageously, a formulation comprising poly(oxazolidone) derivative and a radical/cationic polymerization initiator (e.g. a photo-initiator) is biocompatible.

FIGURES

Figure 1 represents the hemolysis rate in red blood cells after 3 h of direct contact with PU and PHOx/Net discs, assessed by absorbance measurements in the supernatants.

Figure 2 represents the generated nicotinamide adenine dinucleotide calculated from the lactate dehydrogenase activity of human platelets that adhered to PU and PHOx/Net surfaces after 2 h of incubation, assessed by a colorimetric assay.

Figure 3 represents the clotting endpoint times of platelet-poor plasma after 2 h of direct contact with PU and PHOx/Net discs.

Figure 4 represents the metabolic activity of human fibroblasts (HFF-1) after 24 h of indirect contact with PU and PHOx/Net discs, assessed by a fluorescence (resazurin) assay.

Figure 5 represents the metabolic activity of human fibroblasts (HFF-1) after 7 days of indirect contact with PU and PHOx/Net discs, assessed by a fluorescence (resazurin) assay.

Figure 6 represents the metabolic activity of human fibroblasts (HFF-1) after 24 h of direct contact with PU and PHOx/Net discs, assessed by a fluorescence (resazurin) assay.

Figure 7 represents the metabolic activity of human fibroblasts (HFF-1) after 7 days of direct contact with PU and PHOx/Net discs, assessed by a fluorescence (resazurin) assay.

Figure 8 represents the measurement of the time needed for the bacterial activity to reach its peak in a population of *S. aureus* adhered on PU, Silicone and PHOx discs.

Figure 9 represents the measurement of the time needed for the bacterial activity to reach its peak in a population of *S.*

*epidermidis* adhered on PU, Silicone and PHOx discs.

EXAMPLES

**Example 1. Synthesis of polv(hvdroxv-oxazolidone)s**

The synthesis of poly(hydroxy-oxazolidone)s (PHOx) is performed by polyaddition between an α-alkylidene bis(cyclic carbonate) and a polymeric diamine, as shown in the overview below.

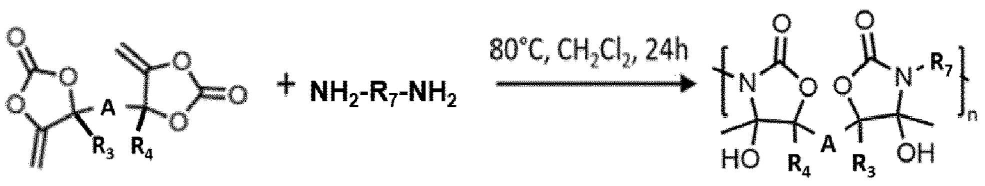


The α-alkylidene bis(cyclic carbonate) compounds used in example 1 are as follows (BisaCC1 (left) and BisaCC2 (right)):

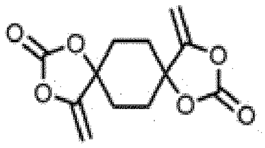

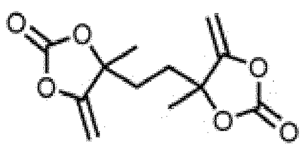

The polymeric diamines used in example 1 are as follows (PPG-$NH_2$ (left) and PDMS-$NH_2$ (right)):

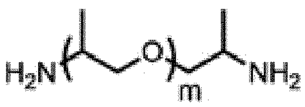

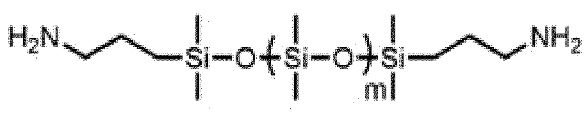

Synthesis of poly(hydroxy-oxazolidone)s

Poly(hydroxy-oxazolidone)s (PHOx) were synthesized as follows. A bis(α-alkylidene cyclic carbonate) (BisaCC1, 2.016 g, 8 mmol, 1 eq.) was weighed in a 100 mL round flask and magnetically stirred with 8 mL of dry $CH_2Cl_2$. Poly(dimethylsiloxane) bis(3-aminopropyl) terminated (PDMS-$NH_2$, Sigma-Aldrich, average Mn = 2,500 g/mol, 20 g, 8 mmol, 1 eq.) was then added under nitrogen atmosphere with a syringe and the solution was stirred 24 h at 80 °C. The poly(hydroxy-oxazolidone)s synthesized starting from PDMS-$NH_2$ with a viscosity of 20-30 cSt (≈ 1000 g/mol from ABCR) and/or from BisaCC2 were prepared according to the same adapted protocol, as well as the poly(hydroxy-oxazolidone) synthesized starting from BisaCC1 and BisaCC2 (0.5 molar eq. of each), and the PPG-based poly(hydroxy-oxazolidone) s, for which the polypropylene glycol) bis(2-aminopropyl ether) (PPG-$NH_2$, Sigma-Aldrich, with an average Mn of 400 or 2,000 g/mol) was purified by three successive azeotropic distillations in dry toluene followed by a vacuum drying prior to synthesis. All polymers were then analyzed by NMR and FTIR.

PHOx1 (precursor BisaCC1 and PPG-NH2 400 g/mol): 1H-NMR (400 MHz, DMSO-d6) δ (ppm) = 5.94 (s, 1H), 3.66 - 3.32 (m, 12H), 1.92 (s, 1H), 1.68 (d, 3H), 1.36 - 1.20 (m, 6H), 1.08 - 0.97 (m, 9H).

PHOx6 (precursor BisaCC2 and PDMS-NH2 2500 g/mol): 1H-NMR (400 MHz, CDCl3) δ (ppm) = 3.12 (s, 2H), 1.96 - 1.19 (m, 10H), 0.46 (s, 2H), 0.17 - 0.00 (s, 117H).

Below are the reaction schemes for the 4 different types of poly(hydroxy oxazolidone) compounds according to the invention as synthesized in example 1:

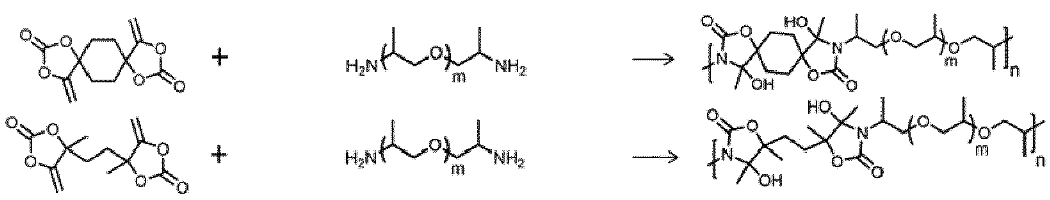

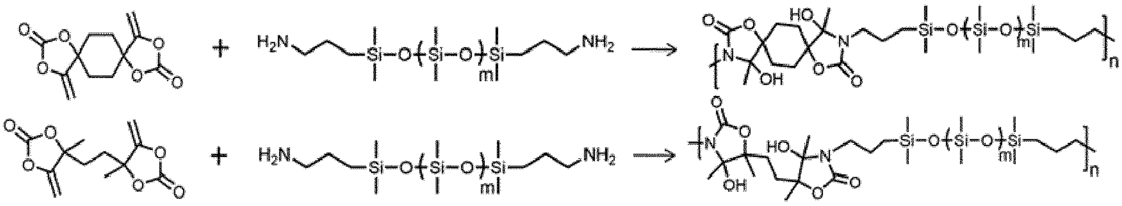

Table 1 below presents the properties of poly(hydroxyoxazolidone compounds synthesized. Different characterization methods were used to evaluate the properties. Fourier transformed infrared spectroscopy (FTIR). Fourier transformed infrared spectra were collected with a Nicolet IS5 spectrometer (Thermo Fisher Scientific) using a diamond attenuated total reflectance (ATR) device. The spectra were obtained in transmission/ATR mode as a result of 32 spectra in the range 4000 - 500 cm-1.

Gel permeation chromatography (GPC). The apparent number-average molecular weight (Mn) of the PPG-based polymers were determined by GPC in DMF with LiBr (2.17 g/L) at a temperature of 55 °C (flow rate of 1 mL/min) with a PSS SECcurity2 autosampler liquid chromatograph calibrated with a PS standard and equipped with three columns (PSS gram 1000 Å (x2), 30 Å), a precolumn, a dual absorbance detector (Waters 2487) and a refractive index detector (Waters 2414). The PDMS-based polymers were determined by GPC in chloroform at a temperature of 35 °C (flow rate of 1 mL/min) using an isocratic pump (VE 1122, Viscotek), a set of two PLgel 5 μm MIXED-C ultrahigh efficiency columns, a Shodex SE 61differential refractive index detector, and a variable wavelength ultraviolet (UV) detector (Spectra 100, Spectra-Physics).

Equilibrium water content measurements (EWC). The equilibrium water contents of the materials were measured after immersion of each sample (previously weighed) in water at room temperature for 24 h. The EWCs were determined by calculating an average of at least three values, obtained by the following equation:

$$\text{Swelling degree} = (W_{s,w} - W_d)/W_d \times 100\%$$

where $W_{s,w}$ is the weight of the swollen sample in water and Wd the weight of the dried sample before swelling in water.

Swelling measurements. The swelling degrees of the networks were calculated by immersing each sample (previously weighed in dry state) in $CHCl_3$ at room temperature and by calculating an average of at least three values, determined by the following equation:

$$\text{Swelling degree} = (W_s - W_d)/W_d \times 100\%$$

where $W_s$ is the weight of the swollen sample in $CHCl_3$ (once the mass is stable) and Wd the weight of the dried sample before swelling.

Gel fraction measurements. The gel fractions of the networks were calculated by comparing the weight of a sample in dry state before immersion in $CHCl_3$ (before swelling) and the weight of a sample after immersion for 24 hours in $CHCl_3$ at room temperature (swelling) and drying at 120 °C overnight to remove $CHCl_3$. They were determined by calculating an average of at least three values, obtained by the following equation:

$$\text{Gel content} = W_{d,f}/W_d \text{ x } 100\%$$

Where $W_{d,f}$ is the weight of the sample dried at 120 °C overnight after immersion in $CHCl_3$ and $W_d$ is the weight of the dried sample before swelling.

Thermogravimetric analyses (TGA) were carried out using a TGA 2 model by Mettler Toledo (weight precision of 0.005% and resolution of 1 μg). Samples were placed under nitrogen atmosphere (20 mL/min) and underwent a first heating from 30 to 100 °C with a heating ramp of 20 °C/min, followed by an isothermal step at 100 °C for 10 min, and a second heating from 100 to 500 °C with a heating ramp of 10 °C/min.

Mechanical properties were measured at a temperature of 25 °C on a Q800 dynamic mechanical analyzer (DMA) from TA Instruments using the DMA controlled force mode. Rectangular-shape samples of 25×5×0.5 $mm^3$ were tested with a chosen starting distance of 7 mm between the two clamps. For each measurement, a starting equilibrium of 5 min was applied at 25 °C, before applying to the sample a force with a ramp of 0.05 MPa/min until its break. The Young's modulus (E), the stress at break (o) and the elongation at break (ε) of the samples were calculated by averaging at least three reproductive values. Samples were analyzed in the dry and in hydrated state (after immersion of sample in water for 24h).

**Table 1a**. Properties of poly(hydroxy-oxazolidone)s.

| Code | BisaCC | Diamine | Molar mass (g/mol) |
|---|---|---|---|
| **PHOx1** | BisaCC1 | PPG-$NH_2$ 400 | ≈ 12,500 |
| **PHOx2** | BisaCC1 | PPG-$NH_2$ 2,000 | ≈ 20,000 |
| **PHOx3** | BisaCC2 | PPG-$NH_2$ 400 | ≈ 13,700 |
| **PHOx4** | BisaCC1 | PDMS-NH2 2,500 | ≈ 25,000 |
| **PHOx5** | BisaCC1 | PDMS- NH2 ≈ 1,000 | ≈ 3,000 |
| **PHOx6** | BisaCC2 | PDMS- $NH_2$2,500 | ≈ 15,700 |
| **PHOx7** | BisaCC1 + BisaCC2 | PDMS- $NH_2$2,500 | ≈ 15,400 |

**Table 1b.** Mechanical Properties of poly(hydroxy-oxazolidone)s.

| Code | Mechanical Properties (dry) | | | Mechanical Properties (wet) | | |
|---|---|---|---|---|---|---|
| | E (MPa) | σ (MPa) | ε (%) | E (MPa) | σ (MPa) | ε (%) |
| **PHOx4** | 2.5 ±0.03 | 0.49 ±0.06 | 52.6 ±11.2 | 1.6 ±0.01 | 0.18 ±0.03 | 23.90 t4.17 |

PHOx4 has a thermoplastic character and its contact angle was measured, so as to evaluate the hydrophobicity. It showed a value of 105 °, hence a value superior to 90 °which shows the hydrophobic nature of the polymer, mainly composed of PDMS. The only other polymer with thermoplastic character, PHOx6, showed a contact angle of 95 °, slightly below the value of PHOx4, which is consistent with the structure of the polymer, the cyclohexyl group being replaced by an ethyl group in the hydroxy-oxazolidone parts. The others viscous PHOxs flowing under the force of gravity, contact angle measurements could not be performed.

The PHOx4 and PHOx6 being the only thermoplastic and solid samples at room temperature, only these two samples could be tested mechanically in the dry and hydrated state. The PHOx4 showed the best mechanical properties out of the two polymers, with a Young's Modulus of 2.5 ± 0.03 MPa, a stress at break of 0.49 ± 0.06 MPa and an elongation at break of 52.6 ± 11.2% in the dry state.

The mechanical properties of the PHOx4, measured after 24h of immersion in water, are slightly impacted compared to the properties in dry state. The Young's Modulus dropped to 1.6 MPa, the stress at break to 0.18 MPa and the elongation at break to 23.9%. This is particularly interesting for applications in a hydrated media such as medical devices in contact with blood. The measured values are comparable to the mechanical values of native tissues reported in the literature.

**Example 2. Dehydration of polv(hvdroxv-oxazolidone)s**

All poly(alkylidene oxazolidone)s (POx) were synthesized by dehydration of their respective poly(hydroxy-oxazolidone)-based precursors as defined and obtained in example 1.

Each sample was put in a Teflon mold before being placed in an oven at 130 °C for 2 h under solvent- and catalyst-free conditions. The viscous samples were then directly analyzed at the output of the oven by $^1$H-NMR, FTIR and DSC.

**POx1** (obtained after dehydration of PHOx1): $^1$H-NMR (400 MHz, DMSO-$d_6$) δ (ppm) = 4.39 (s, 1H), 4.16 (s, 1H), 4.02 (s, 1H), 3.86 (s, 1H), 3.48 (s, 1H), 1.88 (dd, 4H), 1.24 (d, 3H), 1.02 (q, 9H).

**POx6** (obtained after dehydration of PHOx6): $^1$H-NMR (400 MHz, $CDCl_3$) δ (ppm) = 4.06 (dd, 1H), 3.91 (dd, 1H), 3.35 (qq, 2H), 1.72 - 1.51 (m, 4H), 1.38 (s, 3H), 0.51 - 0.42 (m, 2H), 0.17 - 0.00 (s, 117H).

Below the dehydration reaction as performed in example 2:

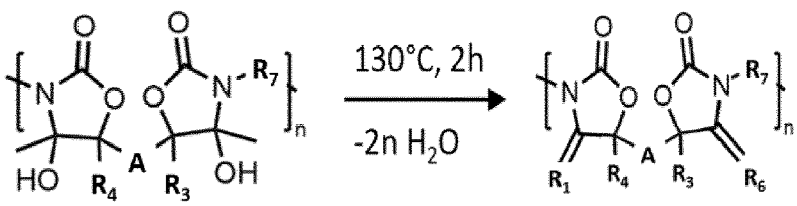

**Table 2.** Properties of poly(alkylidene oxazolidone)s.

| Entry | Code | Precursor | BisaCC | Diamine | Molar mass (g/mol) |
|---|---|---|---|---|---|
| 1 | POx1 | PHOx1 | BisaCC1 | PPG-$NH_2$ 400 | ≈ 11,400 |
| 2 | POx2 | PHOx2 | BisaCC1 | PPG-$NH_2$ 2,000 | ≈ 5,800 |
| 3 | POx3 | PHOx3 | BisaCC2 | PPG-$NH_2$ 400 | ≈ 13,700 |
| 4 | POx4 | PHOx4 | BisaCC1 | PDMS- $NH_2$ 2,500 | ≈ 10,900 |
| 5 | POx5 | PHOx5 | BisaCC1 | PDMS- $NH_2$ ≈ 1,000 | / |
| 6 | POx6 | PHOx6 | BisaCC2 | PDMS- $NH_2$ 2,500 | ≈ 15,700 |
| 7 | POx7 | PHOx7 | BisaCC1 + BisaCC2 | PDMS- $NH_2$ 2,500 | / |

**Example 3. Cross-linking of polyfalkylidene oxazolidone)s**

Poly(alkylidene oxazolidone)s were cross-linked by means of a photothiol-ene reaction with polythiols at room temperature (rt), thanks to the presence of unsaturations on POxs introduced by the dehydration of their PHOx precursors. Any classical photo-initiator suitable for use in photo thiol-ene reactions may be used to accelerate the cross-linking.

All networks (Net) were synthesized according to the same procedure: a poly(alkylidene oxazolidone) was weighed in a small vial (for example POx4, 1 g, 0.74 mmol of unsaturations, 1 eq.) and solubilized in 500 μL of chloroform (≥99.8%, VWR Chemicals). In another vial, the photo-initiator phenylbis(2,4,6-trimethylbenzoyl)phosphine oxide (BAPO, 97%, Sigma-Aldrich, 0.0308 g, 0.07 mmol, 0.1 eq.) was also weighed and dissolved in 100 μL of chloroform by stirring it away from UV light. Trimethylolpropane tris(3-mercaptopropionate) ((≥95%, Sigma-Aldrich, **$SH_3$**, 0.0978 g, 0.24 mmol, 0.33 eq.) was then added to the BAPO solution. The BAPO-thiol solution was then transferred to the first one away from UV light and chloroform was removed under vacuum. The mixture was then poured into flat Teflon molds and irradiated with UV light for 15 min using an Omnicure Series 2000 lamp (200 W, Hg, wavelength of 365 nm). The resulting network was then placed in an oven at 120 °C overnight to remove any remaining traces of chloroform before being analyzed by various techniques. The networks synthesized starting from 2,2'-(ethylenedioxy)diethanethiol (SH2, 95%, Sigma-Aldrich, 0.0671 g, 0.37 mol, 0.5 eq.), pentaerythritol tetrakis(3-mercaptopropionate) (>95%, Sigma-Aldrich, **$SH_4$,** 0.090 g, 0.18 mol, 0.25 eq.) and dipentaerythritol hexakis(3-mercaptopropionate) (>93%, TCI, **$SH_6$**, 0.096 g, 0.12 mol, 0.17 eq.), were prepared following the same adapted protocol. Below the cross-linking of poly(alkylidene oxazolidone)s with polythiols into poly(oxazolidone) networks.

All covalent adaptable networks (Can) were synthesized according to the same procedure: a poly(alkylidene oxazolidone) was weighed in a small vial (for

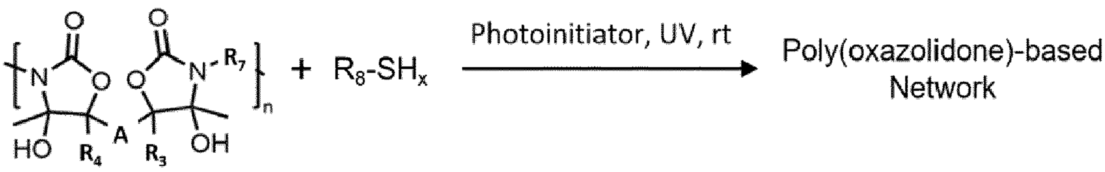


example POx4, 4.122 g, 3.05 mmol of unsaturations, 1 eq.) and solubilized in 3 mL of chloroform (≥99.8%, VWR Chemicals). In another vial, the catalyst methanesulfonic acid (MSA, ≥99.0%, Sigma-Aldrich, 0.0141 g, 0.15 mmol, 0.05 eq.) and trimethylolpropane tris(3-mercaptopropionate) ((≥95%, Sigma-Aldrich, **$SH_3$**, 0.398 g, 1.00 mmol, 0.33 eq.) were also weighed and dissolved in 1 mL of chloroform by stirring it. The MSA-thiol solution was then transferred to the first one, magnetically stirred for 15 min, before being heated at 40-50°C for 2 min in order to initiate the crosslinking. The mixture was then placed in an oven, under vacuum at 100 °C overnight to remove any remaining traces of chloroform and pursue the crosslinking before being analyzed by various techniques. The networks synthesized starting from 2,2'-(ethylene-dioxy)diethanethiol (**$SH_2$**, 95%, Sigma-Aldrich, 0.2728 g, 1.5 mmol, 0.5 eq.), pentaerythritol tetrakis(3-mercaptopropionate) (>95%, Sigma-Aldrich, **$SH_4$,** 0.3656 g, 0.75 mmol, 0.25 eq.) and dipentaerythritol hexakis(3-mercaptopropionate) (>93%, TCI, **$SH_6$**, 0.3915 g, 0.5 mmol, 0.17 eq.), were prepared following the same adapted protocol. Below the cross-linking of poly(alkylidene oxazolidone)s with polythiols into poly(oxazolidone) covalent adaptable networks.

Suitable examples of polythiols ($R_8$-$SH_x$) used in example 3 are the di-thiols ($SH_2$), tri-thiols ($SH_3$), tetra-thiols ($SH_4$) and hexa-thiols ($SH_6$) as shown below:

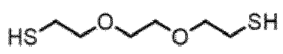

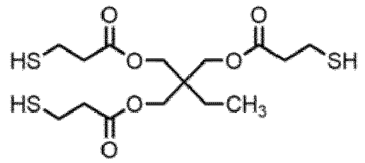

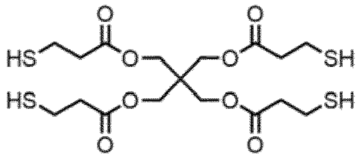

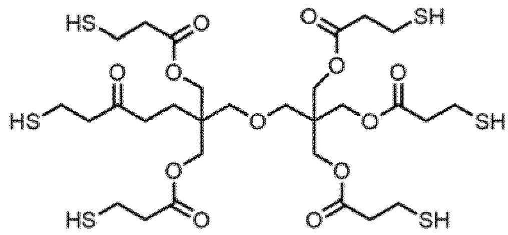

**Table 3.** Properties of poly(oxazolidone) networks.

| Code | | Polythiol . | Swelling in $CHCl_3$ (%) | Gel Content (%) | EWA | Contact angle (°) |
|---|---|---|---|---|---|---|
| **Net1** | POx1 | SH3 | N/A | N/A | N/A | 49±3 |
| **Net2** | POx2 | SH3 | - | - | - | - |
| **Net3** | POx3 | SH3 | - | - | - | |
| **Net4** | POx4 | SH3 | 539.9 ±87.5 | 98.9 ±1.5 | 2.9 ±0.9 | 103 ±2 |
| **Net5** | POx5 | SH3 | N/A | N/A | 14.6 | 79 |
| **Net6** | POx6 | SH3 | - | - | ±1.5 | ±3 |
| **Net7** | POx7 | SH3 | - | - | - | - |
| **Net8** | POx4 | SH2 | 705.6 ±4.3 | 66.5 ±5.8 | 3.2 ±0.8 | 94 ±2 |
| **Net9** | POx4 | SH4 | 697.2 ±31.6 | 83.6 ±4.8 | 2.4 ±0.5 | 109 ±0.5 |
| **Net10** | POx4 | SH6 | 836.4 ±39.5 | 71.8 ±2.1 | 4.0 ±0.3 | 94 ±1 |
| **Can1** | POx4 | SH2 | - | - | - | - |
| **Can2** | POx4 | SH3 | - | - | 5.9 ±1.5 | 95 ±1 |
| **Can3** | POx4 | SH4 | 615.3 ±21.7 | - | 6.0 ±3.0 | 94 ±1 |
| **Can4** | POx4 | SH6 | 1044.8 ±38.2 | - | 5.8 ±1.4 | 95 ±1 |

*- Could not be measured / too viscous / dissolves in $CHCl_3$.*
*Max. referring to machine maximum reached before sample breakage.*
*EWA referring to equilibrium water absorption*

**Table 4.** Mechanical properties of poly(oxazolidone) networks and covalent adaptable networks.

| Code | Mechanical Properties (dry) | | | Mechanical Properties (wet) | | |
|---|---|---|---|---|---|---|
| | E (MPa) | σ (MPa) | ε (%) | E (MPa) | σ (MPa) | ε (%) |
| **Net1** | 8.5 ±0.2 | Max. | Max. | N/A | N/A | N/A |
| **Net2** | - | - | - | - | - | - |
| **Net3** | - | - | - | - | - | - |
| **Net4** | 5.60 ±0.25 | 0.85 ±0.14 | 44.51 ±6.90 | 4.99 ±1.48 | 0.59 ±0.19 | 39.31 ±14.4 |
| **Net5** | 69.6 ±10.9 | 1.73 ±0.68 | 9.07 ±0.93 | 1.37 ±0.40 | 0.29 ±0.03 | 124.08 ±3.70 |
| **Net6** | - | - | - | - | - | - |
| **Net7** | - | - | - | - | - | - |
| **Net8** | 3.74 ±0.02 | 0.66 ±0.01 | 46.06 ±1.30 | 1.06 ±0.20 | 0.21 ±0.07 | 47.23 ±2.15 |
| **Net9** | 6.46 ±0.40 | 0.78 ±0.12 | 24.39 t4.92 | 2.93 ±0.68 | 0.65 ±0.01 | 36.35 ±5.58 |
| **Net10** | 5.42 ±1.00 | 1.36 ±0.22 | 74.94 ±10.00 | 3.79 ±0.80 | 0.74 ±0.21 | 45.95 ±4.78 |
| **Can1** | - | - | - | - | - | - |

(continued)

| Code | Mechanical Properties (dry) | | | Mechanical Properties (wet) | | |
|---|---|---|---|---|---|---|
| | E (MPa) | σ (MPa) | ε (%) | E (MPa) | σ (MPa) | ε (%) |
| **Can2** | 0.79 ±0.06 | 0.39 ±0.03 | 89.21 ±4.22 | 0.76 ±0.04 | 0.27 ±0.01 | 64.60 ±2.75 |
| **Can3** | 1.49 ±0.2 | 0.39 ±0.07 | 4998 ±3.47 | 0.79 ±0.09 | 0.21 ±0.02 | 47.92 ±6.21 |
| **Can4** | 1.10 ±0.14 | 0.41 ±0.02 | 85.61 ±5.28 | 0.98 ±0.36 | 0.20 ±0.07 | 49.61 ±5.28 |
| *- Could not be measured/ Too viscous/dissolves in chloroform.* | | | | | | |

The network formed starting from a PPG type amine (Net1, 49 °) showed a much lower contact angle than those containing PDMS segments (> 79 °). These values are in line with the hydrophilicity of these soft segments, PPG being more hydrophilic than PDMS.

After dehydration, all polymers became viscous, and therefore tensile tests could not be performed on the POxs. Once cross-linked however, the measurement of the mechanical properties of the formed networks could be carried out again.

The PPG-based network Net1 showed a Young's Modulus of 8.5 ± 0.2 MPa (dry state) and reached the limitations of the machine in terms of elongation without breaking.

Regarding the PDMS-based networks, the Net4, formed with $SH_3$, showed dry mechanical property values of 5.60 ± 0.25 MPa (E), 0.85 ± 0.14 (σ) and 44.51 ± 6.90% (ε), which demonstrates the flexibility of these elastomers.

Interestingly, once hydrated, the networks did not show a drastic decrease in mechanical property values, the Young's Modulus being 4.99 MPa, the stress at break 0.59 MPa, and the elongation at break 39.31 %. These measurements demonstrate the low impact of water on the mechanical properties of the network.

This is particularly well-suited for application in medical devices that are in contact with liquid, such as for example body liquids, for example blood.

The Net5, formed with the shorter PDMS precursor of ≈ 1000 g/mol (instead of 2500 g/mol for Net4) presented very high values of E (69.6 MPa) and σ (1.73 MPa), along with a lower ε (9.07%), probably due to the higher cross-linking density in these networks, the masses between the cross-linking nodes being shorter.

When compared together, the photo-crosslinked networks formed from the same precursor (PHOx4), i.e., Net8 (with SH2), Net4 (with SH3), Net9 (with SH4) and Net10 (with SH6), have mechanical properties which follow the same tendencies. Indeed, The Young's Modulus and the stress at break increase with the number of thiol functionalities (from SH2 to SH4), while the elongation at break decreases. An exception can be seen for the network formed with SH6 (Net10), that has lower values than those of Net9 (with SH4), which can be explained by the fact of reaching a certain limit of functionality, beyond which the probability of encounter between the 6 thiol functions of the polythiol SH6 and 6 alkene functions becomes very low in this viscous solution. The final crosslinking density would then actually be lower than that obtained with the SH4 polythiol, which would explain this reduction in mechanical properties.

Regarding the mechanical properties of these networks in the wet state, after immersion for 24h in water, their values are less impacted than in the case of the thermoplastic PHOx4, probably due to the crosslinking of the chains, which limits the impact and insertion of water in the networks. As for the mechanical properties of Net10 measured in the dry state, those of Net10 measured in wet state follow the same tendency, being once again lower than those of Net9. All these values measured in the hydrated state stay however quite high and well-suited for biomedical applications, as in medical devices that are in contact with liquid, such as for example body liquids, for example blood.

The same tendencies are observed with the covalent adaptable networks, as the Young's Modulus increases along with the elongation at break which decreases from the Can2 (formed with SH3) to the Can3 (formed with SH4), in both the dry and hydrated states. Again, the same observations can be made for the covalent adaptable network formed with SH6 (Can4), as for the photo-crosslinked network formed with SH6, as its mechanical properties are lower than Can3 (with SH4), due to a lower crosslinking density coming from the too high polyfunctionality of the crosslinking agent and the high viscosity of the solution.

In all the *in vitro* hemo/biocompatibility and antibacterial tests, medical grade PU (Carbothane™) was used as a reference material. The PU, as well as the synthesized polymers (PHOx/Nets), were cut into 5-mm diameter discs. These discs were rinsed with NaCl 0.9% (w/v) for 72 h (refreshing the NaCl solution every day) at 37 °C under mild agitation (100 rpm). Both surfaces of the discs were sterilized under UV (15 minutes of exposure to each side). For the antibacterial testing, an additional reference of medical grade Silicone was used.

## Example 4. *In vitro* hemocompatibility: interactions with blood components as tested by hemolysis test (erythrocytes)

**Hemolysis test (erythrocytes).** Fresh human blood was collected from healthy donors into citrated tubes (3.8% sodium citrate), and the first tube was discarded to avoid tissue factor effects. Whole blood (WB) was centrifuged at 150xg for 15 min and platelet-rich-plasma (PRP) was removed. Red blood cells (RBCs) were re-suspended in phosphate buffered saline (PBS, Gibco, Thermo Fisher Scientific, USA) and centrifuged at 800xg for 10 min. RBCs were washed for another 2 times and a final ratio of 1:7 RBCs/PBS was used for hemolysis tests. PU and the synthesized polymers were incubated with the RBCs/PBS for 3 h at 37 °C in an orbital shaker at 100 rpm. Using distilled water (dHaO), a solution of RBCs/d$H_2$O (1:7) was prepared and used as positive control of hemolytic effect. After 3 h of incubation, the RBCs solutions were centrifuged at 800xg for 15 min, the supernatants were transferred to a 96-well polystyrene plate, and the absorbance was measured (a ≈ 545 nm) using a micro-plate reader. Absorbance values were compared to the positive and negative controls, and hemolysis rates were displayed in percentage.

The Standard Practice for Assessment of Hemolytic Properties of Materials describes 3 different categories: hemolytic materials, with a percentage of hemolysis greater than 5%; slightly hemolytic materials, with a hemolytic index between 5% and 2%; and non-hemolytic materials, with the hemolysis percentage below 2% Standard Practice for Assessment of Hemolytic Properties of Materials, ASTM F756-00, (2010), 5). According to these criteria, all the tested polymers (PHOx4, Net1, Net4 and Net5) were non-hemolytic, similar to the PU reference (see **Figure 1**).

## Example 5. In vitro hemocompatibility: interactions with blood components as tested by lactate dehydrogenase activity (platelet adhesion)

Platelet adhesion on the polymer surfaces was evaluated in lactate dehydrogenase activity (LDH) assays, which allow the quantification of the generated NADH. WB was centrifuged at 150xg for 15 min, PRP was collected, and platelets were counted. The desired concentration (250 000 platelets/μL) was obtained through dilution in platelet-poor plasma (PPP). Polymer discs were incubated with platelets for 2 h at 37 °C in an orbital shaker at 100 rpm. After 2 h of incubation, adherent platelets were washed with PBS and subsequently lysed with Triton-X100 (Merck, 1%). Lactate dehydrogenase (LDH) activity in the lysates was measured using a colorimetric assay kit from Sigma-Aldrich. LDH reduces nicotinamide adenine dinucleotide (NAD) to NADH, specifically detected by absorbance measurements (λ ≈ 450 nm). Absorbance values were compared to a standard curve (standards included in the commercial kit) and statistically significant differences between PU and PHOx/Nets were calculated through one-way ANOVA with Tukey's multiple comparisons test.

The mean value of generated NADH on PU surfaces was higher than on PHOx4, and similar to what was found on Net1/Net4/Net5 discs (see Figure 2). The results indicate very low levels of platelet adhesion on the surface of PHOx4, indicating lower thrombogenicity of this polymer than medical grade PU. The other polymers Net1, Net 4, Net5 induced similar platelet adhesion as the PU reference.

## Example 6. *In vitro* hemocompatibility: interactions with blood components as tested by coagulation test (clotting time)

Coagulation test was performed as follows. PPP (CRYOcheck Pooled Normal Plasma (Cryopep, France) was thawed at 37 °C for 5 minutes and then incubated with the polymer discs. After 2 h of incubation, human plasma clotting time was measured through a non-activated partial thromboplastin time (NaPTT) test. NaPTT reagent (NODIA) was added to the PPP samples and incubated for 1 minute at 37 °C. A solution of calcium chloride (0.025 M) was added to the prewarmed samples, and clotting endpoints were measured using a Stago STart 4 apparatus.

After incubating human plasma with PU and PHOx/Net discs, similar mean clotting times were found (see **Figure 3**). Among the tested polymers, PHOx4 showed the longest clotting times. Overall, none of tested polymers induced more contact activation of the coagulation than the PU reference.

## Example 7. *In vitro* biocompatibility: interactions with human fibroblasts with indirect contact (extracts)

**Cell line and culture conditions.** *In vitro* biocompatibility assays were performed using Human Foreskin Fibroblasts (HFF-1). Cells were expanded in Dulbecco's Modified Eagle's Medium (DMEM, Gibco, Thermo Fisher Scientific, USA) supplemented with 10% volume/volume (v/v) fetal bovine serum (FBS, Gibco, Thermo Fisher Scientific, USA) and 1% v/v penicillin/streptomycin (Pen/Strep) at 37 °C, in a humidified atmosphere, containing 5% v/v $CO_2$. Medium was refreshed every two days. When reaching 90% confluence, cells were rinsed with 5 mL of PBS (37 °C) and detached from culture flasks using 2 mL of Trypsin-EDTA solution.

**Indirect Contact (extracts).** The cytocompatibility of the synthesized polymers (PHOx/Nets) was initially

evaluated using the indirect contact assay, by incubation of the cells with extracts of the materials, which were prepared as described in ISO 10993-12:2004. Briefly, to prepare the extracts, sterile 5-mm polymer discs were incubated with DMEM for 24 h at 37 °C in an orbital shaker at 100 rpm. Cells were seeded in 96-well plates at density of $1 \times 10^5$ cells/mL and kept in culture for 24 h at 37 °C with 5% v/v $CO_2$. After 24 h, when a cell monolayer was formed, the materials' extracts were added, according to ISO 10993-5:2009(E), and the cells were incubated for other 24 h or 7 days. A solution of Triton (X-100) 0.1% v/v was used as positive control of *in vitro* cytotoxicity.
According to ISO 10993-5:2009(E) - Biological evaluation of medical devices - Part 5: Tests for in vitro cytotoxicity, (2009), a threshold of 70% in the metabolic activity percentages (when comparing to CTL -) should be set to consider a material as non-cytotoxic. After 24 h of incubation with polymers extracts, the results revealed percentages higher than 70% for the synthesized PHOx/Nets, while for PU the mean metabolic activity was slightly lower than this threshold (see **Figure** 4). This indicates that the leaching products of the PHOx/Nets discs extracted for 24 h were not cytotoxic for HFF-1 cells. After 7 days, the percentages were higher than 70% for all the tested polymers (see **Figure 5**), including for PU, indicating that the cells fully recovered.

## Example 8. *In vitro* biocompatibility: interactions with human fibroblasts with direct contact

**Direct Contact.** Cells were seeded in 48-well plates at density of $1 \times 10^5$ cells/mL and kept in culture for 24 h at 37 °C with 5% v/v $CO_2$. After 24 h, when a cell monolayer was formed, the sterile polymer discs (PHOx/Nets) were added to each well, and the cells were incubated for other 24 h or 7 days. A solution of Triton (X-100) 0.1% v/v was used as positive control of *in vitro* cytotoxicity.
After 24 h of direct contact with polymer discs, the results revealed percentages higher than 70% for all the tested samples - synthesized PHOx/Nets and medical grade PU (see **Figure 6**). When this direct contact was extended to 7 days, the metabolic activity was still higher than 70% for the synthesized PHOx/Nets, while for PU the percentage decreased considerably, approaching the limit of 70% (see **Figure 7**). With both indirect and direct contact assays, it was shown that PHOx/Nets are biocompatible *in vitro.*

**Measurement of the metabolic activity.** Metabolic activity of the fibroblasts was quantified through a resazurin-based assay after 24 h or 7 days of indirect and direct contact with PHOx/Nets extracts and discs, respectively. For that, culture medium was removed from the wells and fresh media containing 10% v/v resazurin (10-oxido-7-oxophenoxazin-10-ium-3-olate, Stemcell Technologies, Canada), was incubated with the cells for 4 h at 37 °C with 5% v/v $CO_2$. Afterwards, medium was transferred to a black 96-well plate and the relative fluorescence units (RFUs) were measured ($\lambda_{ex} \approx 530$ nm, $\lambda_{em} \approx 590$ nm) using a micro-plate reader. Results were displayed as a percentage of the negative control (metabolic activity of cells incubated with PHOx/Net extracts/discs relatively to cells in contact with DMEM alone).

## Example 9. Antibacterial testing: adhesion of *Staphylococci*

**Bacterial strains and culture conditions.** Bacterial adhesion assays were performed using *Staphylococcus aureus* (*S. aureus*) (ATCC BAA-1556) and *Staphylococcus epidermidis* (*S. epidermidis*) (ATCC 35984). For each bacterial strain, 2 colonies were picked and cultured overnight in 5 mL of Tryptic Soy Broth (TSB, MilliPore, Thermofisher, USA) at 37 °C with agitation (190 rpm).

**Anti-adhesive properties of the surfaces.** The antibacterial potential of the synthesized PHOx was evaluated by incubation of the polymer discs with bacterial suspensions. Sterile 5-mm polymer discs of PHOx and controls (PU and Silicone) were incubated with a bacterial inoculum of $10^5$ CFUs/mL in TSB for 2 h at 37 °C in static conditions. After the 2-h adhesion step, polymer discs were rinsed with 500 μL of PBS, so unattached or poorly attached bacteria could be removed. The discs with adherent bacteria were transferred to calWell optical grade sterile plastic inserts (SYMCEL) and 100 μL of fresh TSB was added to each sample. The plastic inserts were then placed inside titanium cups which are closed with titanium lids, and the metallic ensemble was incubated in the calScreener equipment for 24 h under static conditions at 37°C. This allows the measurement of calorimetry, an ultrasensitive approach based on the first law of thermodynamics to measure smallest heat changes in minimum sample volumes.

**Measurement of the bacterial activity.** Bacterial activity was measured through detection of the produced heat, a by-product of biological processes. Heat is directly related to growth and heat production rate to the metabolic fluxes. Maximum heat flow and time to peak were measured for both bacterial strains.

The monitoring of adherent *Staphylococcus aureus* on the polymer discs (PU, Silicone and PHOx4) revealed that the mean time to peak was longer for PHOx than for PU and silicone, the later yielding the shortest time to peak **(Figure 8).** These data show that the initial number of bacteria on PHOx discs (after removing non-attached bacteria from the previous 2-h pre-adhesion step) was lower than on the two reference polymers, therefore indicating that PHOx displays bacterial anti-adhesion properties compared to PU and silicone. This behavior compared to the Silicone reference is surprising given the fact of the major proportion of PDMS in the PHOx4.

The measurement of bacterial activity on adherent *Staphylococcus epidermidis* confirmed the previous observations, with similar results: the longest time to peak was again measured on PHOx4 surfaces, while the shortest was found on Silicone discs (see **Figure 9).** These data indicate that the anti-adhesive effect is not exclusive towards *S. aureus* but also seen when using *S. epidermidis.*

## Claims

**1.** A poly(hydroxy oxazolidone) compound corresponding to formula (I) or (II)

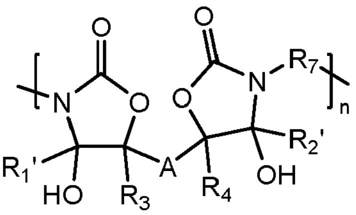

(I)

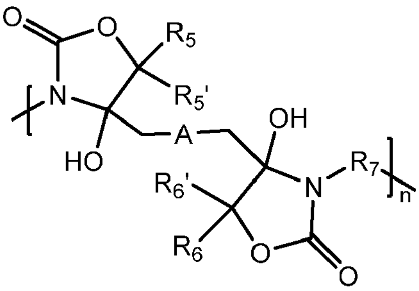

(II)

wherein

- Rr, $R_{2'}$ independently of one another each denote a C1 to C20 alkyl group, preferably a C1 to C10 alkyl group, more preferably a C1 to C3 alkyl group, most preferably a methyl group, and
- $R_3$, $R_4$ independently of one another each denote hydrogen or a C1 to C20 alkyl group, preferably a C1 to C10 alkyl group, more preferably a C1 to C3 alkyl group, most preferably a methyl group, and
- $R_5$, $R_{5'}$, $R_6$, $R_6$, independently of one another each denote hydrogen or a C1 to C20 alkyl group, preferably a C1 to C10 alkyl group, more preferably a C1 to C3 alkyl group, most preferably a methyl group, or an aryl group, preferably a phenyl group, and
- A is an organic linking group preferably selected from a linear, branched, saturated, unsaturated, cyclic and/or non-cyclic aliphatic, aromatic or araliphatic hydrocarbon group, preferably having 1 to 20, more preferably 1 to 12, most preferably 1 to 8 carbon atoms and optionally further containing one or more functional groups selected from ether, ester, amide, amine, carboxyl, sulfur, halogen and/or urethane, and
- Optionally A, $R_3$ and $R_4$ in formula (I) are linked to each other to form a cyclic structure, and
- Optionally $R_5$ and $R_{5'}$, and/or $R_6$ and $R_{6'}$, in formula (II) are respectively linked to each other to form a cyclic structure, and
- $R_7$ is a polymeric chain selected from a polysiloxane or polyether, and
- n is an integer from 2 to 100, preferably 2 to 50, more preferably 2 to 25, most preferably 2 to 15.

**2.** The poly(hydroxy oxazolidone) compound according to claim 1 wherein $R_7$ is a polymeric chain selected from a polysiloxane, preferably Polydimethylsiloxane (PDMS) having a molecular weight having a molecular weight in the range 900 - 30000 g/mol, preferably in the range 1500 - 10000 g/mol, more preferably in the range 2000 - 5000 g/mol, most preferably in the range 2000 - 3000 g/mol or a polyether selected from a polyalkylene glycol, preferably selected from polypropylene glycol (PPG) or polyethylene glycol (PEG) and combinations thereof, having a molecular weight in the range 230 - 10000, preferably in the range 230 - 5000 g/mol, more preferably in the range 300 - 4000 g/mol, most preferably in the range 350 - 3000 g/mol.

**3.** A formulation for preparing poly(hydroxy oxazolidone) compounds, said formulation comprising

- at least one multifunctional cyclic carbonate having at least two terminal $\alpha$-alkylidene cyclic carbonate groups (compound A) according to formula (III) or (IV)

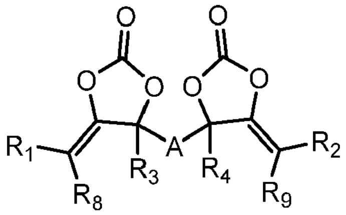 (III) 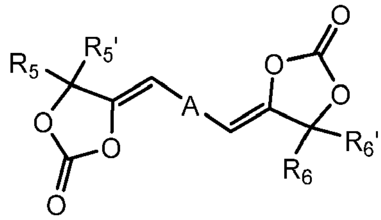 (IV)

Wherein

- $R_1$, $R_2$, $R_8$, $R_9$ independently of one another each denote hydrogen or a C1 to C20 alkyl group, preferably a C1 to C10 alkyl group, more preferably a C1 to C3 alkyl group, most preferably a hydrogen, and
- $R_3$, $R_4$ independently of one another each denote hydrogen or a C1 to C20 alkyl group, preferably a C1 to C10 alkyl group, more preferably a C1 to C3 alkyl group, most preferably a methyl group, and
- $R_5$, $R_{5'}$, $R_6$, $R_{6'}$ independently of one another each denote hydrogen or a C1 to C20 alkyl group, preferably a C1 to C10 alkyl group, more preferably a C1 to C3 alkyl group, most preferably a methyl group, or an aryl group, preferably a phenyl group, and
- A is an organic linking group selected from a linear, branched, saturated, unsaturated, cyclic and/or non-cyclic aliphatic, aromatic or araliphatic hydrocarbon group, preferably having 1 to 20, more preferably 1 to 12, most preferably 1 to 8 carbon atoms and optionally further containing one or more functional groups selected from ether, ester, amide, amine, carboxyl, sulfur, halogen and/or urethane, and
- Optionally A, $R_3$ and $R_4$ in formula (III) are linked to each other to form a cyclic structure, and
- Optionally $R_5$ and $R_{5'}$, and/or $R_6$ and $R_{6'}$ in formula (IV) are respectively linked to each other to form a cyclic structure.

- at least one multifunctional amine (compound B) wherein the multifunctional amine is composed of a polymeric chain having at least two primary amine groups at the end of said polymeric chain and wherein the polymer chain is selected from a polysiloxane or a polyether.

**4.** The formulation according to claim 3, wherein the multifunctional amine is a polymeric diamine containing a polymeric chain with at least two primary amine groups at the end of said polymeric chain, preferably the multifunctional amine is corresponding to the formula $NH_2$-$R_7$-$NH_2$ wherein $R_7$ is composed of a polymeric chain selected from a polysiloxane or a polyether.

**5.** The formulation according to claim 3 or 4, wherein the multifunctional amine is corresponding to the formula $NH_2$-$R_7$-$NH_2$ wherein $R_7$ is selected from Polydimethylsiloxane (PDMS) having a molecular weight having a molecular weight in the range 900 - 30000 g/mol, preferably in the range 1500 - 10000 g/mol, more preferably in the range 2000 - 5000 g/mol, most preferably in the range 2000 - 3000 g/mol or selected from a polyether, preferably selected from a polyalkyleneglycol, more preferably selected from Polypropyleneglycol (PPG) and/or Polyethylene glycol (PEG) having a molecular weight in the range 230 - 10000 g/mol, preferably in the range 230 - 5000 g/mol, more preferably in the range 300 - 4000 g/mol, most preferably in the range 350 - 3000 g/mol.

**6.** The formulation according to any of foregoing claims 3-5 wherein the multifunctional cyclic carbonate (compound A) is selected from at least one of following compounds:

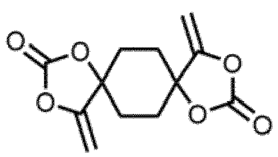 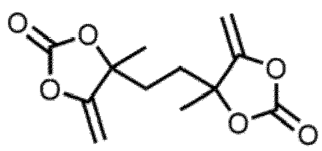 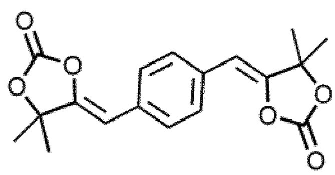

**7.** A process for forming the poly(hydroxy oxazolidone) compound according to claims 1-2 said process comprising at least a polyaddition step between the multifunctional cyclic carbonate (compound A) and the multifunctional amine (compound B) in the formulation according to any of foregoing claims 3-6.

**8.** A process for forming poly(alkylidene oxazolidone) compounds, said process comprising at least the step of

dehydrating the poly(hydroxy oxazolidone) compounds according to claims 1-2 and wherein the obtained poly(alkylidene oxazolidone) compounds are according to formula (V) or (VI).

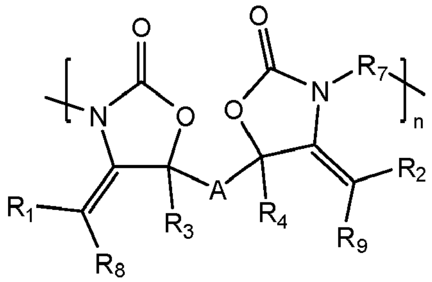 (V) 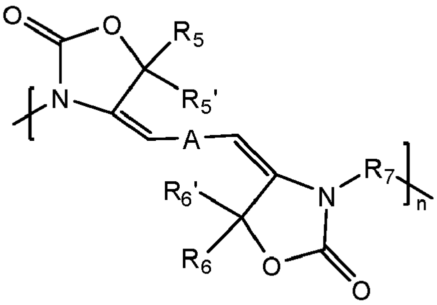 (VI)

Wherein

- $R_1$, $R_2$, $R_8$, $R_9$ independently of one another each denote hydrogen or a C1 to C20 alkyl group, preferably a C1 to C10 alkyl group, more preferably a C1 to C3 alkyl group, most preferably a hydrogen, and
- $R_3$, $R_4$ independently of one another each denote hydrogen or a C1 to C20 alkyl group, preferably a C1 to C10 alkyl group, more preferably a C1 to C3 alkyl group, most preferably a methyl group, and
- $R_5$, $R_{5'}$, $R_6$, $R_{6'}$ independently of one another each denote hydrogen or a C1 to C20 alkyl group, preferably a C1 to C10 alkyl group, more preferably a C1 to C3 alkyl group, most preferably a methyl group, or an aryl group, preferably a phenyl group, and
- A is an organic linking group selected a linear, branched, saturated, unsaturated, cyclic and/or non-cyclic aliphatic, aromatic or araliphatic hydrocarbon group, preferably having 1 to 20, more preferably 1 to 12, most preferably 1 to 8 carbon atoms and optionally further containing one or more functional groups selected from ether, ester, amide, amine, carboxyl, sulfur, halogen and/or urethane, and
- Optionally A, $R_3$ and $R_4$ in formula (V) are linked to each other to form a cyclic structure, and
- Optionally $R_5$ and $R_{5'}$, and/or $R_6$ and $R_{6'}$ in formula (VI) are respectively linked to each other to form a cyclic structure, and
- $R_7$ is a polymeric chain selected from a polysiloxane or a polyether, and
- n is an integer from 2 to 100, preferably 2 to 50, more preferably 2 to 25, most preferably 2 to 15.

**9.** A poly(alkylidene oxazolidone) compound obtained according to the process of claim 8.

**10.** A cross-linkable formulation for forming a network of poly(oxazolidone) compounds, said formulation comprising:

- At least one poly(alkylidene oxazolidone) compound according to claim 9
- At least one polythiol corresponding to formula $R_8$-$SH_x$ wherein $R_8$ is an organic group selected from a C2-C20 alkyl group, and optionally further containing one or more functional groups selected from ether ester amide, amine, carboxyl, sulfur, halogen and/or urethane and x is an integer between 2-8, preferably between 2 and 6
- Optionally a radical and/or cationic polymerization initiator, preferably selected from a photopolymerization initiator and/or a thermal polymerization initiator.

**11.** The formulation according to claim 10 wherein polythiol is selected from following compounds:

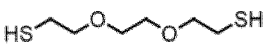 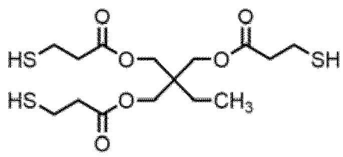 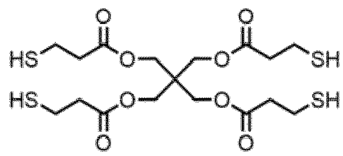

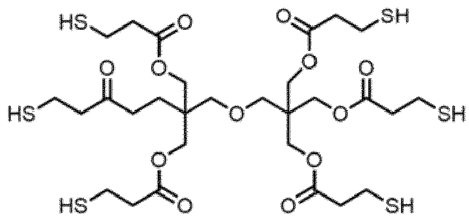

**12.** The formulation according to claim 10 or 11 wherein the radical polymerization initiator is a photopolymerization initiator selected from alkylphenone-based photopolymerization initiator, α-aminoalkyl ketone-based photopolymerization initiators, phosphine oxide-based photopolymerization initiators and combinations thereof and/or wherein the polymerization initiator is a cationic polymerization initiator preferably selected from acids such as methane sulfonic acid, sulfuric acid, p-toluene sulfonic acid and combinations thereof.

**13.** A process for making cross-linked poly(oxazolidone)s, said process comprising at least the steps of providing a cross-linkable formulation according to any of claims 10-12 and polymerizing the poly(alkylidene oxazolidone) compounds with the polythiol and optionally in the presence of a suitable radical and/or cationic polymerization initiator to form a cross-linked poly(oxazolidone).

**14.** The process according to claim 13 wherein the step of polymerizing the poly(alkylidene oxazolidone) compounds with the polythiol is a radical thiol-ene polymerization reaction performed at room temperature and with irradiation with light, preferably light selected from UV, red and/or visible light and the initiator is selected from a radical photo polymerization initiator.

**15.** Cross-linked poly(oxazolidone)s made according to any of claims 13-14.

**16.** Use of the poly(hydroxy oxazolidone) compound according to any of claims 1-2 in a medical device.

**17.** Use of the cross-linked poly(oxazolidone)s of claim 15 in a medical device.

**18.** A medical device wherein the device contains at least partly the poly(hydroxy oxazolidone) compounds according to any of claims 1-2 and the poly(hydroxy oxazolidone) compounds in said device are preferably at least partly provided by means of coating, injecting molding, extrusion, extrusion-based additive manufacturing techniques.

**19.** A medical device wherein the device contains at least partly the cross-linked poly(oxazolidone)s according to claim 15 and the cross-linked poly(oxazolidone)s in said device is preferably at least partly provided by means of coating, irradiation in a mold or light-based additive manufacturing techniques/stereolithography.

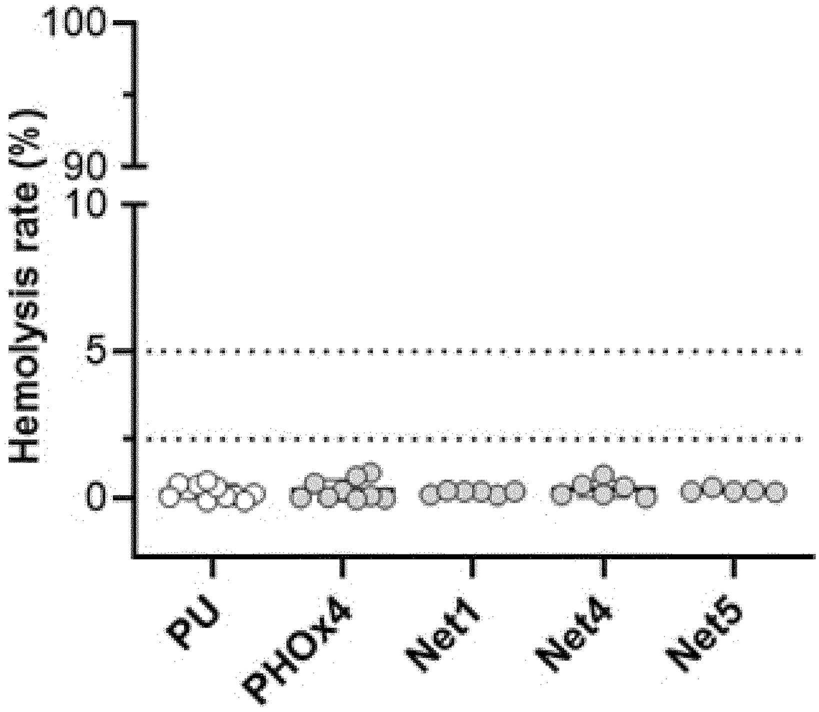


Figure 1

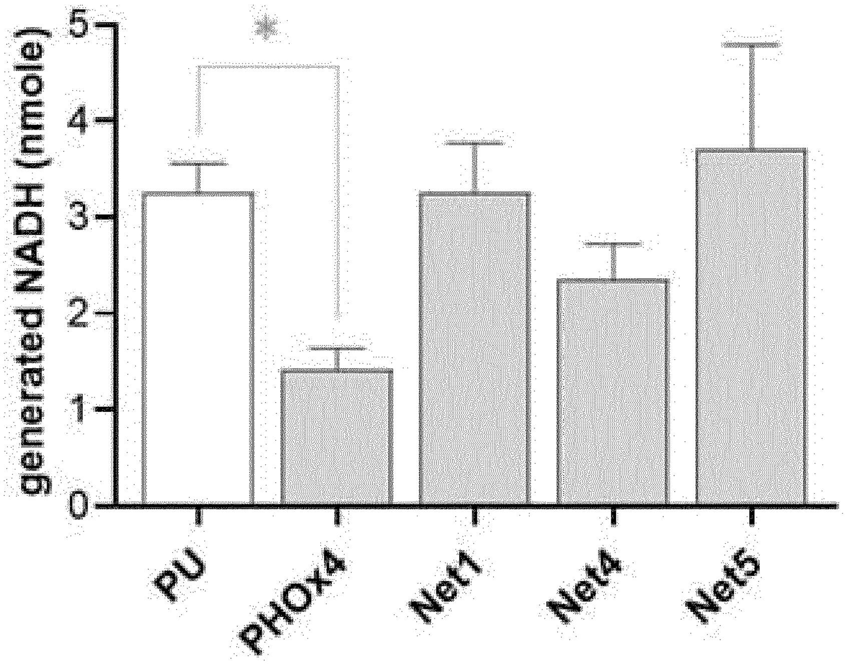


Figure 2

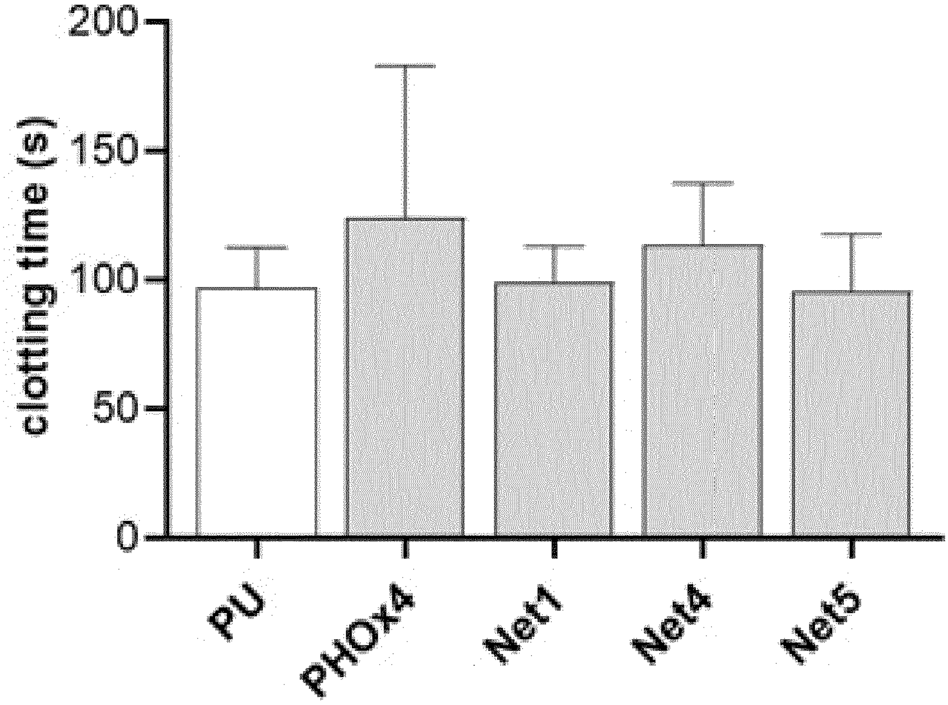


Figure 3

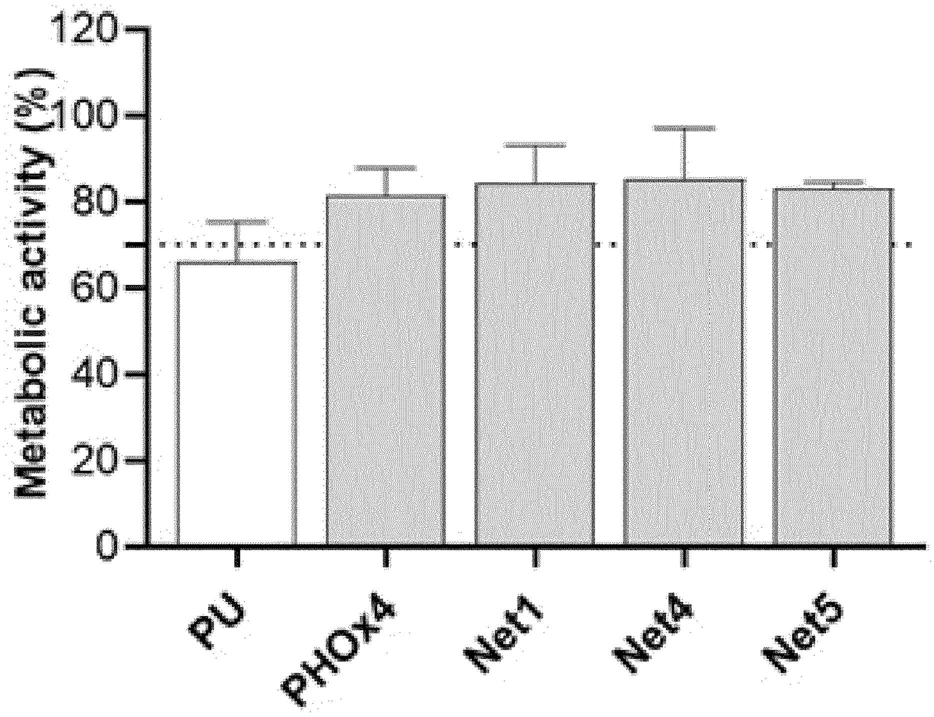


Figure 4

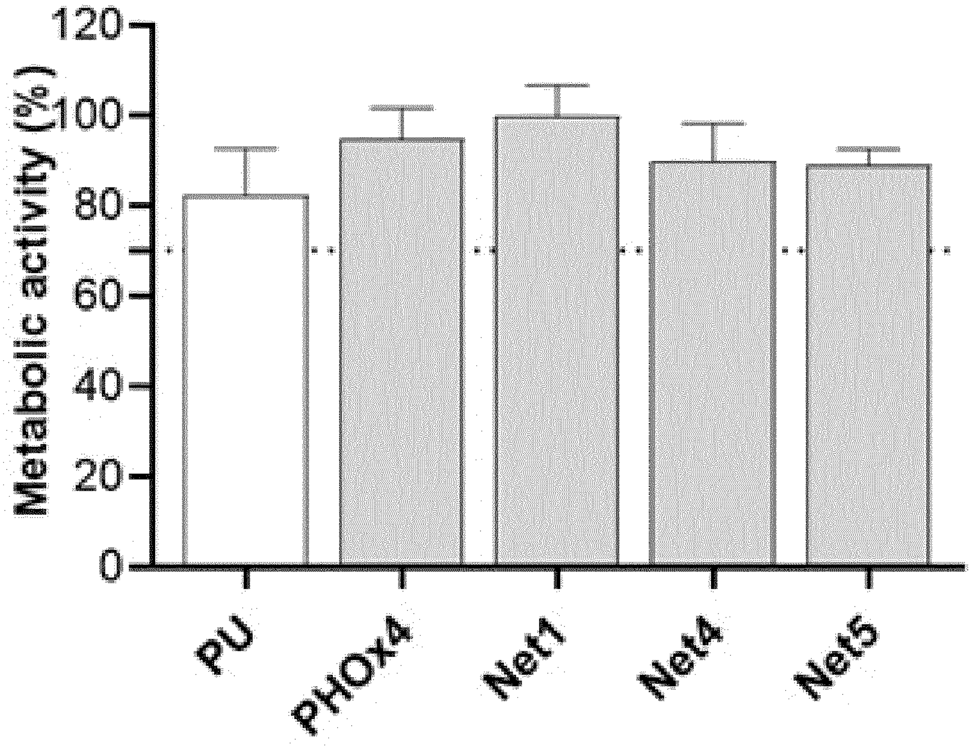


Figure 5

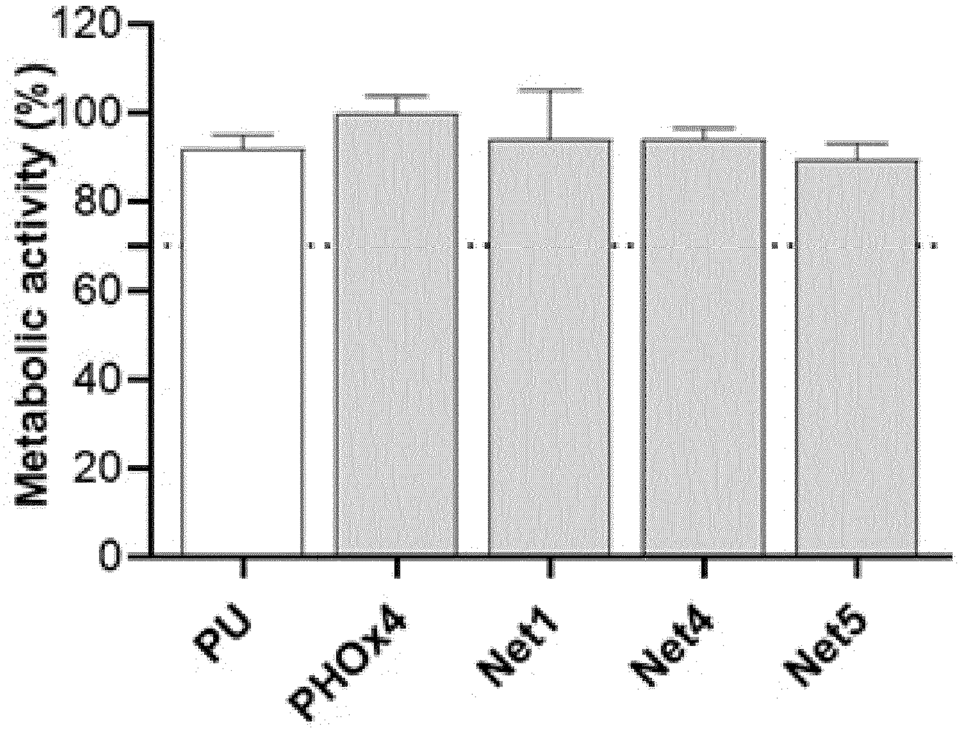


Figure 6

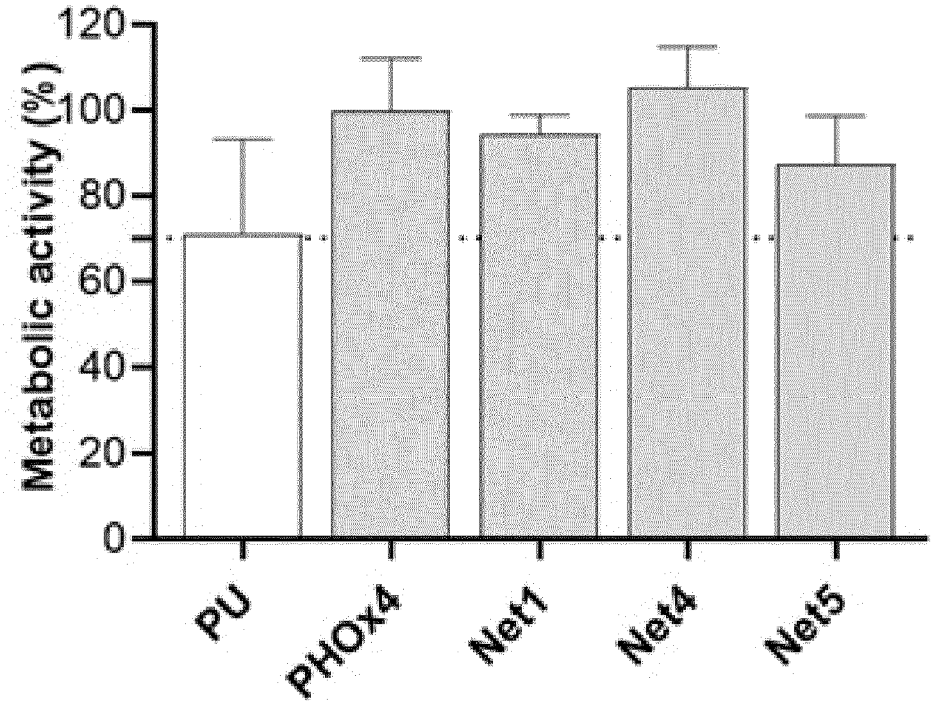


Figure 7

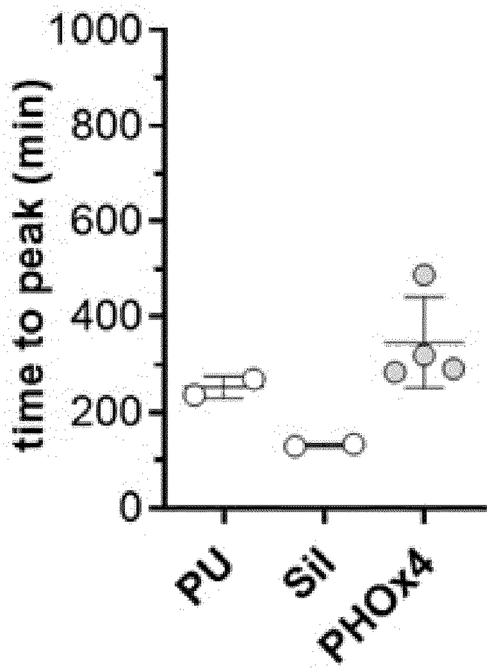


Figure 8

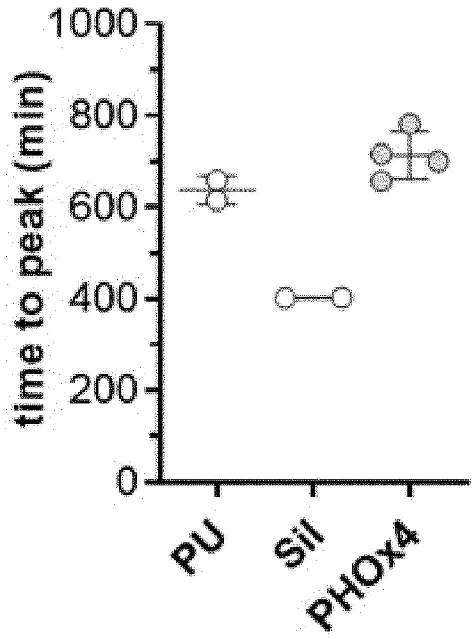


Figure 9

Europäisches Patentamt
European Patent Office
Office européen des brevets

# EUROPEAN SEARCH REPORT

Application Number
EP 23 20 4802

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HABETS THOMAS ET AL: "Advancing the Synthesis of Isocyanate-Free Poly(oxazolidones)s: Scope and Limitations", MACROMOLECULES, vol. 53, no. 15, 28 July 2020 (2020-07-28), pages 6396-6408, XP055938654, US ISSN: 0024-9297, DOI: 10.1021/acs.macromol.0c01231 Retrieved from the Internet: URL:https://pubs.acs.org/doi/pdf/10.1021/acs.macromol.0c01231> * schemes 1-2, p.6397 * * "conclusions"; page 6406 - page 6407 * | 1-19 | INV. C08G71/04 A61F2/06 A61F2/24 B33Y70/00 |
| X | WO 2013/001096 A1 (DSM IP ASSETS BV [NL]; PARAKKA JAMES [NL] ET AL.) 3 January 2013 (2013-01-03) * page 3, line 14 - line 25; claims 1-14 * * page 4, line 13 - line 15 * | 1-19 | |
| | | | TECHNICAL FIELDS SEARCHED (IPC) |
| A | WO 2022/180235 A1 (UNIV LIEGE [BE]; CENTRE HOSPITALIER UNIV DE LIEGE [BE]) 1 September 2022 (2022-09-01) * claims 1-16 * | 1-19 | C08G A61F B33Y |
| A | WO 2015/158718 A1 (MONTANUNIVERSITÄT LEOBEN [AT]) 22 October 2015 (2015-10-22) * claims 1-47 * | 1-19 | |

The present search report has been drawn up for all claims

1

| Place of search | Date of completion of the search | Examiner |
|---|---|---|
| Munich | 28 March 2024 | Scheuer, Sylvie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 20 4802

This annex lists the patent family members relating to the patent documents cited in the above-mentioned European search report.
The members are as contained in the European Patent Office EDP file on
The European Patent Office is in no way liable for these particulars which are merely given for the purpose of information.

28-03-2024

| Patent document cited in search report | Publication date | Patent family member(s) | Publication date |
|---|---|---|---|
| WO 2013001096 A1 | 03-01-2013 | AU 2012277738 A1 | 16-01-2014 |
| | | CA 2840611 A1 | 03-01-2013 |
| | | CN 103649099 A | 19-03-2014 |
| | | CN 105949466 A | 21-09-2016 |
| | | EP 2726489 A1 | 07-05-2014 |
| | | JP 5944987 B2 | 05-07-2016 |
| | | JP 2014527031 A | 09-10-2014 |
| | | JP 2016196644 A | 24-11-2016 |
| | | US 2014206830 A1 | 24-07-2014 |
| | | WO 2013001096 A1 | 03-01-2013 |
| WO 2022180235 A1 | 01-09-2022 | CN 116368168 A | 30-06-2023 |
| | | EP 4298151 A1 | 03-01-2024 |
| | | JP 2024510363 A | 07-03-2024 |
| | | WO 2022180235 A1 | 01-09-2022 |
| WO 2015158718 A1 | 22-10-2015 | AU 2015248910 A1 | 17-11-2016 |
| | | BR 112016023872 A2 | 15-08-2017 |
| | | CA 2979581 A1 | 22-10-2015 |
| | | CN 106456831 A | 22-02-2017 |
| | | EP 3131952 A1 | 22-02-2017 |
| | | EP 3540013 A1 | 18-09-2019 |
| | | ES 2746548 T3 | 06-03-2020 |
| | | ES 2925242 T3 | 14-10-2022 |
| | | KR 20170010309 A | 26-01-2017 |
| | | US 2017051112 A1 | 23-02-2017 |
| | | WO 2015158718 A1 | 22-10-2015 |
| | | ZA 201607572 B | 25-09-2019 |

EPO FORM P0459

For more details about this annex : see Official Journal of the European Patent Office, No. 12/82